# EUROPEAN PATENT SPECIFICATION

(11) **EP 1 427 821 B1**
(45) Date of publication and mention of the grant of the patent: **20.06.2007**
(21) Application number: 02806590.2
(22) Date of filing: 15.08.2002
(51) Int. Cl.: C12N 15/00, C12N 15/09, C12N 15/86, C12Q 1/00, C12Q 1/68, C12P 21/06, C07H 21/04

(54) **BIOSENSOR FOR SMALL MOLECULE ANALYTES**
BIOSENSOR FÜR KLEINMOLEKULARE ANALYTEN
BIOCAPTEUR POUR PETITS ANALYTES MOLECULAIRES

(30) Priority: 20.08.2001 US 313714 P
(43) Date of publication of application: 16.06.2004
(73) Proprietor: Regenesis Bioremediation Products, San Clemente, CA 92673 (US)
(72) Inventor: LAING, Lance, G., Belmont, MA 02478 (US)
(74) Representative: Howard, Paul Nicholas
(86) International application number: PCT/US2002/026105
(87) International publication number: WO 2003/062786

(56) References cited:
- EP-A- 0 611 828
- WO-A-01/61037
- US-A- 4 921 788
- US-B1- 6 329 160
- US-B1- 6 436 651

## Description

### Field of the Invention

The invention relates to the field of biosensors and, more particularly to a biosensor for detecting the presence of molecules and small compounds, particularly metal ions and metal ion complexes, using a competitive molecular binding assay.

### Background of the Invention

The term biosensor refers generally to a class of devices that recognize a desired compound (analyte) in a sample and selectively generate a signal which can be resolved to determine the concentration of the compound within the sample. One desirable characteristic of many biosensors is their ability to distinguish a specific analyte without the need for separation or isolation. For example, biosensors can detect the presence of a particular compound within a blood or water sample directly thereby eliminating the need for lengthy or complex purification steps to recover the analyte of interest.

Biosensor technology can be further characterized by the coupling of a biological recognition system with an electrochemical transducer so as to produce an electrical signal or impulse which is used in analyte concentration determination. Typical classifications for electrochemical transducers used in conventional biosensors include potentiometric, amperometric, or optical-based transducer mechanisms.

In potentiometric-based sensor devices, the accumulation of charge density at the surface of an electrode is measured and is representative of the concentration of analyte to which the biosensor is exposed. One example of a potentiometric-based biosensor is the ion-selective field-effect transistor (ISFET) sensors that may be used for the detection of ions such as iron or a gas such as carbon dioxide or oxygen.

In an amperometric sensor, electrons that are exchanged between a biological system and an electrode generate a current which may be monitored to determine the concentration of analyte within the sample. Amperometric sensors are commonly employed in blood glucose and ethanol sensors, as well as other devices that monitor compounds of biological significance.

While the aforementioned biosensor devices may be adequate for relatively gross quantitation of analytes within a sample solution, they lack the sensitivity necessary to detect inorganic matter in quantitative trace analysis procedures, and they lack specificity as many interfering species may cause inaccurate charge-related errors. Quantitative trace analysis of inorganic matter is useful in identifying elements or compounds such as arsenic that may be present in a sample at very low concentrations and are of significance to investigators. Such analytical methods have applicability in many fields and are particularly relevant in areas such as public health and environmental toxicology.

More recently, optical biosensors have been developed which utilize a light wave detection methodology designed to monitor changes in analyte concentration by coupling the change in the analyte concentration with a change in the characteristics of the detected light waves. A particular class of optical sensor devices which have been described for use in biosensors include surface plasmon resonance (SPR) sensors. Surface plasmon resonance is an optical phenomenon which is observed at a metal film-liquid interface where total internal reflection (TIR) of light occurs. During the process of total internal reflection on the surface plasmon resonance sensor, a component of the incident light, termed the evanescent wave, excites molecules in close proximity to the interface. In the case of the optical sensor, the evanescent wave energy is absorbed by the metal film layer of the sensor resulting in a change in the reflected angle and light intensity.

Based upon the aforementioned SPR principle, changes in the refractive index of a solution can be assessed and quantified by the optical sensor as changes in reflected light intensity. The BIAcore biosensor (Pharmacia, Sweden) has been previously described and incorporates SPR sensor technology to quantify globular proteins in a solution. A drawback imposed by this device, apart from its high cost, is that it lacks the ability to perform inorganic matter detection and quantitation directly. Such a device also lacks portability. Furthermore, this system is not suitable for direct quantification of trace amounts of analyte, in the low parts per billion range. Additionally, this system is limited with respect to the types of molecules that can be detected and may not be suitable for detection of multiple different types of analytes using the same sensing device.

Thus, in the analysis of inorganic matter there is a need for a detection system which can accurately perform quantitation procedures in a rapid and reliable manner. Using this system a biosensor device should desirably employ a highly specific detection method capable of identifying trace quantities of atomic or ionic matter in complex mixtures and should be useable with both liquid and gaseous samples. Another desirable characteristic which is lacking in many existing biosensors, including those based on atomic adsorption and colorimetric methods, is the ability to detect a variety of different analytes such as proteins, nucleic acids, organic molecules, inorganic molecules, and the like using a single biosensor platform with limited modifications.

### Summary of the Invention

Embodiments of this invention include materials (e.g., proteins and DNA oligonucleotides), devices, systems, and methods for detecting and quantifying trace amounts of small molecule analytes, particularly metal ions and metal ion complexes, in environmental or biological samples using constituents or modified constituents of naturally occurring proteins and nucleic acids. This technology possesses properties of high sensitivity, capable of detecting specific analytes in the low parts per billion range and may be used to identify numerous types of materials in a variety of different states or conditions.

In one aspect, the invention comprises a biosensor used to detect and quantify toxic inorganic materials, such as arsenic, using an evanescent wave-employing device upon which a competitive binding surface is formed. The sensor surface is coated with a first class of molecules whose sequence or structure exhibits properties of reversible binding to a second class of molecules. The quantity of the second class of molecules bound to the first class of molecules on the sensor surface give rise to a characteristic change in refractive index which is measured by determining a difference and/or rate of change in the resonance angle of free oscillating electrons in the sensor surface. These resonance angle changes result in changes in quantity, intensity or direction of refracted light directed from the sensor surface. Detection and quantitation of an analyte is therefore based on a change in characteristics of the refracted light, which is measured as a function of an electronic detection signal generated by evanescent wave production in the sensor device and subsequent alteration in the reflected light intensity.

In one embodiment, detection of an analyte occurs when the sensor surface is exposed to the analyte, which exhibits properties of competitive binding to specific proteins or peptides present on the sensor surface. As the analyte binds to the protein, the protein concentration or state of binding on the chip surface is altered and a change in the reflected light intensity is observed and measured.

Thus, the present invention provides in one embodiment a system for detecting the presence of a metal compound in a sample. This system comprises (i) an isolated protein that specifically binds a metal compound; (ii) an isolated nucleic acid containing a specific binding sequence which is bound specifically by the protein, wherein binding of the protein to the metal compound causes a conformational change in the protein that releases it from binding to the nucleic acid; and (iii) a detection system that indicates release of the protein from the nucleic acid. The protein undergoes an allosteric change upon binding the metal compound. In a specific embodiment of the system, the protein is a bacterial DNA-binding regulatory protein encoded by a metal-response operon and the nucleic acid is a DNA containing a specific binding sequence from the metal-response operon promoter that is specifically bound by the metal compound.

In various embodiments, the metal compound can be Ag, As, Ba, Cd, Cr, Hg, Pb, or Se, and more particularly Ag, As, Cd, Cr, Hg, or Pb. Other metal compounds of interest include Be, Cu, Ni, Ti, and Zn; Al, Co, Fe, Mg, Mn, K, Na, and V are also of interest in Superfund cleanup sites. In addition, the invention permits detection of useful metals in prospecting, such as Ag, Cu, Fe, Al, Au, Ti, Pt, etc. Radionuclides like U, Pu, Tc, Np, Cs, and I can also be detected with a biosensor of the invention. A system and method for detecting As are exemplified.

The invention also provides a biosensor device based on the metal compound detection system. In such a device, either the protein or the nucleic acid is bound to a solid phase support. In a specific embodiment, the solid support is contained in a flow cell that permits flow of liquid over the solid support. In a specific example exemplified infra, the solid support comprises a metal film that forms an interface with liquid, wherein the detection system is a surface plasmon resonance system. Preferably such a device maintains a stable temperature of the solid support and the sample to minimize the effects of temperature on detection of surface plasmon resonance, e.g., by using a material of high heat capacity. A microprocessor capable of processing changes in plasmon resonance at the surface over time may also be included in the device to facilitate analysis of the binding interactions.

The invention further provides a method of detecting the presence of a metal compound in a sample. This method comprises contacting the sample with a protein that specifically binds a metal compound, which protein is bound to an isolated nucleic acid containing a specific binding sequence for the protein, wherein binding of the protein to the metal compound causes a conformational change in the protein that releases it from binding to the nucleic acid. After this contacting occurs, the method provides for detecting release of the protein from the nucleic acid.

In a specific embodiment, the protein comprises a sequence of a bacterial DNA-binding regulatory protein encoded by a metal-response operon sufficient to bind DNA and the metal. In another embodiment, the nucleic acid is a DNA containing a sequence that is modified to differ by at least one nucleotide from a specific binding sequence from a metal-response operon promoter that is specifically bound by the protein, and which DNA is specifically bound by the protein with different binding constants than the unmodified sequence. In a further embodiment, both a modified protein and a modified nucleic acid are used. Such modifications permit expansion of the dynamic range of the system, or lead to increased sensitivity and specificity, or both, as required by the system.

Also provided in this invention is ArsR protein comprising an amino acid sequence that is at least 90% identical to the amino acid sequence of amino acids 1-117, more particularly 1-97, of SEQ ID NO:2, which ArsR protein binds to a nucleic acid sequence selected from the group consisting of SEQ ID NOS: 3, 4, 5, 6, 7, 8, 9, and 10. Such a protein may further contain a purification handle or tag, such as a hexahistidine sequence. The invention similarly provides DNA oligonucleotides, particularly double-stranded oligonucleotides, comprising an ArsR binding sequence. Such an oligonucleotide has a sequence that differs by no more than three bases or base pairs from a sequence selected from the group consisting of SEQ ID NOS: 3, 4, 5, 6, 7, 8, 9, and 10.

These and other aspects, advantages, and novel features of the invention will become apparent upon reading the following detailed description and upon reference to the accompanying drawings.

### Brief Description of the Drawings

In the drawings, same elements have the same reference numerals in which:
Figure 1 illustrates a block diagram for a competitive coupling biosensor device.
Figure 2 illustrates an exemplary competitive binding layer present on the biosensor surface used to detect analyte molecules.
Figure 3 illustrates the equilibrium dissociation factors associated with competitive binding in an arsenic biosensor.
Figure 4 illustrates a method for preparing the competitive coupling biosensor device.
Figure 5 illustrates a method for qualitative and quantitative assessment of the presence of an analyte using the biosensor device.
Figures 6A-C illustrate one embodiment of a competitive coupling biosensor device.
Figures 7A-C illustrate another embodiment of the competitive coupling biosensor device.
Figures 8A-C illustrate yet another embodiment of the competitive coupling biosensor device.
Figures 9A-C illustrate a further embodiment of the competitive coupling biosensor device.
Figures 10A-B illustrate graphs which measure the effect of ligand concentration on the competitive binding complex.
Figure 11 illustrates a process for detecting a target analyte using the biosensor device.
Figure 12 illustrates a perspective view of an apparatus.
Figure 13 illustrates a top view of the apparatus illustrated in Figure 12.
Figure 14 illustrates a cross sectional view of Figure 13 cut along the line indicated.
Figure 15 illustrates a bottom view and Figure 16 a frontal view of the apparatus.
Figure 17 illustrates a bottom perspective view of a flow cell.
Figure 18 illustrates a top perspective view of the flow cell illustrated in Figure 17.

### Detailed Description

The present invention advantageously provides a biosensor that rapidly and effectively detects small compounds, for example metals and metal ion complexes, as exemplified below with arsenic. A biosensor by definition assembles a biological moiety (of various organic nature-carbohydrates, proteinaceous material, nucleic acids, or lipids) with an analytical detection process or instrument for sensing the presence of specific trace quantities of another material(s). Ions and very small molecules are by their chemical nature difficult to identify and quantify using traditional spectroscopic or other classical chemical methods. Metal/metal ion complex ions (arsenic, lead, chromium, cadmium) and small molecules, such as, for example, petroleum hydrocarbons (e.g., benzene), gasoline oxygenates (ethers, e.g., MTBE), organic halides (e.g., TCE) and nitroaromatics (e.g., TNT) are a large and growing environmental and health concern. The present invention, which takes advantage of specific binding characteristics of molecules, which may be affected by allosteric changes to one binding partner's conformation, permits accurate detection and quantitation of such small molecules.

In the Example below for the detection of arsenic ions, a system and method has been devised that provides for the accurate and specific quantification of trace amounts of the toxic states of arsenic. The system is specific (no cross reactivity detected to date), sensitive (conservatively below 10 ppb with a probable detection limit of less than Ippb), fast (about 2 minutes), and easy to use (no special knowledge required). Furthermore, by adapting a very small sensor like the "Spreeta^{™}" chip, the invention permits the development of small, handheld biosensors of the invention.

As used herein, the term "system" refers to a combination of (i) a first class of molecules, such as an isolated protein that specifically binds an analyte and a nucleic acid; (ii) a second class of molecules, such as an isolated nucleic acid containing a specific binding sequence which is bound specifically by the protein; and (iii) a detection system that indicates release of the first class of molecules (protein) from the second class of molecules (nucleic acid). In a first embodiment, binding of the first class of molecules (e.g., protein) to the analyte and the second class of molecules (e.g., nucleic acid) is mutually exclusive, such that, for example, release of bound protein occurs in the presence of the analyte. For example, as exemplified *infra,* binding of a protein to a metal compound causes a conformational change in the protein that releases it from binding to a nucleic acid, thus binding to the metal compound and the nucleic acid are mutually exclusive. In an alternative embodiment, binding of the first class of molecule to the analyte may be necessary for binding to the second class of molecule, such that, for example, protein bound to analyte in solution then binds to nucleic acid. Thus, a system of the invention provides for detection of a change in binding of the first class of molecules to the second class of molecules in the presence compared to the absence of the analyte.

In a specific embodiment, the system employs a solid phase support, to which either the nucleic acid or the protein is bound. However, many liquid phase and homogenous detection systems are known, e.g., from the immunoassay field, and these can be readily adapted to the present invention.

Reference made to "protein" and "nucleic acid" (or DNA) herein should not be construed to limit the invention to these preferred embodiments, which are used for ease of understanding (protein-DNA binding being more readily envisioned than, for example, first class of molecule-second class of molecule binding).

The term "protein" is used herein to refer to a polymer of amino acids, and includes full-length wild-type proteins, DNA and analyte-binding domain fragments thereof, as well as variants described below. The term is used herein as equivalent to "polypeptide". While the present invention specifically exemplifies proteins as molecules capable of specific binding to both a small molecule analyte and to a binding partner, such as a nucleic acid, it should not be so limited. Any molecule that is capable of either mutually exclusive binding (as further elaborated below) or cooperative binding (also as elaborated below) can be used in the practice of this invention.

A "nucleic acid" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. As used herein, the term "oligonucleotide" refers to a nucleic acid, generally of at least 10, preferably at least 15, and more preferably at least 20 nucleotides, preferably no more than 100 nucleotides, that contains a specific protein binding site. Generally, oligonucleotides are prepared synthetically, preferably on a nucleic acid synthesizer, but can also be obtained from cellular or cloned DNA and RNA. If synthetic, oligonucleotides can be prepared with non-naturally occurring phosphoester analog bonds, such as thioester bonds, etc. Specific non-limiting examples of synthetic oligonucleotides include oligonucleotides that contain phosphorothioates, phosphotriesters, methyl phosphonates, short chain alkyl, or cycloalkyl intersugar linkages or short chain heteroatomic or heterocyclic intersugar linkages. Most preferred are those with CH₂-NH-O-CH₂, CH₂-N(CH₃)-O-CH₂, CH₂-O-N(CH₃)-CH₂, CH₂-N(CH₃)-N(CH₃)-CH₂ and O-N(CH₃)-CH₂-CH₂ backbones (where phosphodiester is O-PO₂-O-CH₂). US Patent No. 5,677,437 describes heteroaromatic oligonucleoside linkages. Nitrogen linkers or groups containing nitrogen can also be used to prepare oligonucleotide mimics (U.S. Patents No. 5,792,844 and No. 5,783,682). U.S. Patent No. 5,637,684 describes phosphoramidate and phosphorothioamidate oligomeric compounds. Also envisioned are oligonucleotides having morpholino backbone structures (U.S. Patent No. 5,034,506). In other embodiments, such as the peptide-nucleic acid (PNA) backbone, the phosphodiester backbone of the oligonucleotide may be replaced with a polyamide backbone, the bases being bound directly or indirectly to the aza nitrogen atoms of the polyamide backbone (Nielsen et al., Science 1991, 254:1497). Other synthetic oligonucleotides may contain substituted sugar moieties comprising one of the following at the 2' position: OH, SH, SCH₃, F, OCN, O(CH₂)ₙNH₂ or O(CH₂)ₙCH₃ where n is from 1 to about 10; C 1 to C10 lower alkyl, substituted lower alkyl, alkaryl or arylalkyl; Cl; Br; CN; CF₃; OCF₃; O-; S-, or N-alkyl; O-, S-, or N-alkenyl; SOCH₃; SO₂CH₃; ONO₂ ;NO₂; N₃; NH₂; heterocycloalkyl; heterocycloalkaryl; aminoalkylamino; polyalkylamino; substituted silyl; a fluorescein moiety; an RNA cleaving group; a reporter group; an intercalation; a group for improving the pharmacokinetic properties of an oligonucleotide; or a group for improving the pharmacodynamic properties of an oligonucleotide, and other substituents having similar properties. Oligonucleotides may also have sugar mimetics such as cyclobutyls or other carbocyclics in place of the pentofuranosyl group. Nucleotide units having nucleosides other than adenosine, cytidine, guanosine, thymidine and uridine, such as inosine, may be used in an oligonucleotide molecule.

While the present invention exemplifies nucleic acids as the second class of molecules, one of ordinary skill in the art can readily appreciate that other molecules are also capable of specific binding to a first class of molecules, e.g., a protein, either exclusively or cooperatively with an analyte. Such molecules include, but are not limited to (1) peptides and proteins, (2) carbohydrates, (3) other small molecule compounds, and (4) glycans. Thus the invention need not be limited to any particular class of molecule in this respect.

Furthermore, where it is desirable to identify new sequences for a second class of molecules, whether nucleic acid, peptide, carbohydrate, small molecule, etc., various libraries of such molecules, including combinatorial libraries, can be employed to advantage. Alternatively, or as a method for increasing assay efficiency, microarrays of such molecules can be tested for specific binding interactions.

The term "analyte" refers to a small molecule of interest, i.e., for which detection by a biosensor of the invention is desirable. Analytes can be organic or inorganic, or metallo-organic. The term "inorganic material" as used herein includes metallo-organics. Analytes can carry charge, and thus the term includes cations (e.g., metal ions or metal ion complexes), anions (e.g., phosphates and nitrates, which can be found in areas of intensive agricultural activity), salts, neutral species, and the like. The biosensors of the invention are particularly useful for detecting metal compounds.

The term "metal compound" refers to a metal ion, metal ion complex (metallo-organic), or other molecular form of metal ion. Preferably, the metal is an element at position 21 or higher on the periodic table, and includes metalloids (such as As) and certain "non-metallic" elements (e.g., Se). More preferably still, the metal is a toxic heavy metal. Thus, the systems and devices of the invention can be used to detect: As, Cd, Cr, Pb, Hg. The Resource Conservation Recovery Act eight includes: Ag, As, Ba, Cd, Cr, Hg, Pb, Se. Priority pollutant metals (thirteen) are: Sb, As, Be, Cd, Cr, Cu, Pb, Hg, Ni, Se, Ag, Ti, and Zn. The Comprehensive Environmental Response, Compensation, and Liability Act (CERCLA), a.k.a. Superfund Contract Laboratory Program (CLP) list includes 23 metals: Al, Sb, As, Ba, Be, Cd, Ca, Cr, Co, Cu, Fe, Pb, Mg, Mn, Hg, Ni, K, Se, Ag, Na, Tl, V, Zn. The invention further contemplates systems, devices, and methods for detecting metals such as Au, Ag, Al, Cu, Fe, Pt, and Ti. Radionuclides of concern that can be detected with a biosensor of the invention include U, Pu, Tc, Np, Cs, and I. Metal compounds are a class of small molecules that can be detected by the systems, devices, and methods of the invention.

Organic analytes of interest include, but are not limited to, insecticides, herbicides, halogenated hydrocarbons, aromatics, and the like.

A "sample" refers to a material to be tested for the presence of a small molecule, e.g., a metal ion or metal ion complex. Samples include, but are not limited to, groundwater, reservoir water, lakes and rivers, mine tailings, dirt from toxic waste sites, and prospecting ore samples, to mention a few. In a specific embodiment, the sample is a liquid or a liquid extract of the solid to facilitate binding and release interactions of the first and second classes of binding molecules, e.g., protein and nucleic acid.

The term "operon" is used herein in accordance with its ordinary meaning in the art, i.e., to refer to a group of coding sequence under expression control of a single promoter. Operons are usually found in bacterial genomes. In the practice of the present invention, the operon will typically encode a promoter-binding repressor protein (although activator proteins, that bind to the nucleic acid when bound to the analyte of interest are also contemplated) that is sensitive to the presence of a small molecule target, e.g., metal ion or metal ion complex. In repressor systems, this promoter binding protein provides feedback control for expression from the operon, as described below. In one embodiment, the metal-binding protein and DNA from the promoter are isolated from the same operon. Operons are well known for a variety of metal ions, including but not limited to *ars, czc, cad, chr, mer,* and *pbr.* Operons with non-metal binding specificity are also known, such as the *tet* repressor system and the *lac* operon, as well as multidrug resistance operons, e.g., *bmr* of *B. subtilis, nor* and *qac* of *S*. *aureus, emr* of *E. coli, mex* of *P. aeruginosa,* and quorum sensing systems, such as *las* and *rhl* of *P. aeruginosa.* Many of these systems are reversible, that is, they can be made to bind to the operon in the presence rather than the absence of the inducer molecule. A specific example is the *tet* activator mutant.

The following table discloses operon systems suitable for the invention:

| **Element analyte** | **Operon** | **Example Protein** |
|---|---|---|
| Arsenic | *ars* | ArsR, ArsD |
| Cadmium,Zinc, Cobalt | czc, | CzcR, CzcD |
| Nickel | *cnr* & *ncc* | CnrR |
| Cadmium | *cad* | CadC |
| Chromium | *chr* | ChrB |
| Mercury | *mer* | MerR1 & 2, MerD |
| Molybdate | *mod* | ModE |
| Lead | *pbr and pum* | pbrR |
| Silver | *sil* | silR |
| Tellurite | *tel or the* | TelR |
| Zinc | *smt* | SmtB |
| Copper | *cop* & *pco* | CopR, CopS, PcoR, PcoS |

Where an operon is not yet known, or not likely to exist for responding to a specific small molecule of interest, the well-known techniques of directed evolution and gene shuffling (see, e.g., U.S. Patent Nos. 6,420,175, 6,352,842, 6,335,160, 6,319,713, and 6,238,884; PCT Publication No. WO 02/10750; Beste, et al., Proc. Natl. Acad. Sci. USA 1999, 96:1898-1903; Bailey, Nature Biotech, 1999;17:616-618; Reynolds, Proc. Natl. Acad. Sci. USA, 1998, 95:12744-12746; Cane, et al., Biochemistry 1999, 38:1643-1651; Lau, et al., Nature 1994, 370:389-391) provide for development of a protein that binds to a DNA having a specific sequence and to the molecule of interest, such that binding to the molecule of interest is mutually exclusive of binding to the nucleic acid, e.g., induces a conformational change that results in release of the protein from the DNA; or causes binding to the nucleic acid. Additionally, systems could be created using specific metal binding proteins engineered with specific DNA recognition peptide motifs that overlap or impede the same was as repressor proteins such as ArsR does with the metal binding site.

A "biosensor device" is any manufactured machine that employs a system of the invention. Generally, such a device will have an inlet port to permit entry of a liquid sample. In one embodiment, the device will be prepared by first providing a solution containing the soluble binding molecule, which will be permitted to bind to the second molecule, which may be free in the device or, as exemplified below, bound to a solid support. Alternatively, the device can be provided with the first and second binding molecules already reversibly bound to each other, ready to receive sample. In the case where the device detects binding of the protein to the nucleic acid in the presence of analyte, the device provides for admixture of the protein and sample prior to or during introduction to the nucleic acid. Preferably the device will provide for temperature control, whether passively (through use of high specific heat components) or actively (through the use of a heating or cooling element). The biosensor device will also include detectors to detect binding and release of the first and second molecule. Such detectors include optical detectors, e.g., of surface plasmon resonance or fluorescence. Alternatively, electrical, e.g., "DNA wire", type detection systems can be employed to advantage.

In a specific alternative embodiment of the mutually exclusive binding embodiment, the first class of molecule (protein) is contacted with the analyte prior to contact with the second class of molecule (nucleic acid). Referring now specifically to an embodiment in which the first class of molecule is a protein and the second class a nucleic acid (but not being bound thereto), to measure a difference in binding of the protein the device comes with protein already bound to immobilized nucleic acid. The level of binding is determined; this level serves as a control for maximum binding. Then, the protein is washed (under high salt or other fairly harsh conditions) from the nucleic acid, leaving the nucleic acid free to form a complex with protein. Any residual washing buffer is removed from the system so that binding can proceed normally. Prior to contact with the nucleic acid, the protein and analyte sample are mixed together. If there is analyte in the sample, it will retard protein binding to the nucleic acid. This block or delay in binding will be of greater magnitude than would be possible if the analyte were displacing the protein from the nucleic acid. If there were no analyte in the sample, the protein would bind at high levels to the nucleic acid. This embodiment simplifies the detection step, and provides a more robust measurement. Naturally, where cooperative binding is the basis for detecting the presence of analyte, the first class of molecule must be in contact with the analyte and the second class of molecule simultaneously.

Performance of an analytical test using a system and biosensor of the invention can involve control not only of temperature, but also pH, redox potential, salt concentration, and any other variables. Depending on the system involved, the nature of the sample, and the type of analyte, one or more of these factors can disrupt the assay. Moreover, the invention contemplates optimizing one or more of these factors, preferably two or more in combination, to achieve desirable assay sensitivity and specificity.

As used herein, the term "isolated" means that the referenced material is free of components found in the natural environment in which the material is normally found. In particular, isolated biological material is free of cellular components. In the case of nucleic acid molecules, an isolated nucleic acid includes but is not limited to a PCR product, an isolated mRNA, a cDNA, a restriction fragment, or an oligonucleotide. Isolated nucleic acid molecules can be inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated material may be, but need not be, purified. However, for the most sensitive and specific operation of the invention, the isolated material should be purified.

The term "purified" as used herein refers to material that has been isolated under conditions that reduce or eliminate unrelated materials, i.e., contaminants. For example, a purified protein is preferably substantially free of other proteins or nucleic acids with which it is associated in a cell or recombinant expression; a purified nucleic acid molecule is preferably substantially free of proteins or other unrelated nucleic acid molecules with which it can be found within a cell. As used herein, the term "substantially free" is used operationally, in the context of analytical testing of the material. Preferably, purified material substantially free of contaminants is at least 50% pure; more preferably, at least 90% pure, and more preferably still at least 99% pure. Purity can be evaluated by chromatography, gel electrophoresis, immunoassay, composition analysis, biological assay, and other methods known in the art.

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

### Description of a Specific Embodiment

While the following description refers to the Figures, it should be understood that the procedures, reagents, and assay conditions set forth herein could apply to other assay systems as well.

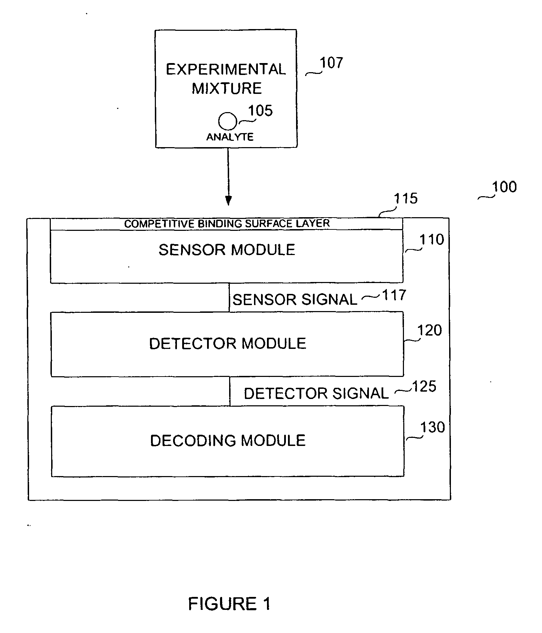

Figure 1 illustrates a block diagram for a biosensor device 100 used in the detection and quantitation of a target analyte 105 from an experimental mixture 107. The biosensor 100 comprises a sensor module 110 having a competitive binding surface (CBS) layer 115 which is responsive to specific analytes 105 based on the composition of the layer 115. The sensor module 110 identifies the presence of the analyte 105 through selective binding with components of the CBS layer 115 to generate a sensor signal 117 which is received by a detector module 120. The detector module 120 then transforms the sensor signal 117 into a detector signal 125, which is received by a decoding module 130 and is used for the purposes of identifying and quantifying the analyte present about the CBS layer 115.

As will be subsequently described in greater detail, the biosensor 100 desirably detects the presence of the analyte 105 comprising molecules to which the CBS layer 115 is exposed in a specific and highly-sensitive manner. The quantity and type of molecules to be detected further serve as a basis for determining the composition of the CBS layer 115. In one aspect, the biosensor device 100 desirably provides increased sensitivity in detecting the analyte 105 through the use of a competitive binding assay wherein the composition of the CBS layer 115 results in the binding of one or more components of the CBS layer 115 with the analyte 105. The resultant binding of the target analyte 105 with components of the CBS layer 115 results in the generation of the resolvable sensor signal 117 which is transformed into a value or identifier representative of the concentration of the analyte 105 in the experimental mixture 107.

One feature of the biosensor 100 device resides in the ability to rapidly acquire qualitative and quantitative information about the composition of the analyte 105 contained in the experimental mixture 107 in a rapid and reproducible manner. Furthermore, the sensitivity of the biosensor 100 is increased by coupling the sensor module detection signal 117 with the detector module which amplifies the signal 117 and subsequently produces the detector signal 125 which can identify very low concentrations of the target analyte 105. A further advantage of the biosensor device 100 is that experimental mixtures 107 of solid, liquid, and gaseous forms may be used in the quantification of the target analyte 105 with the concentration of the analyte 105 present in trace amounts relative to other components in the experimental mixture 107.

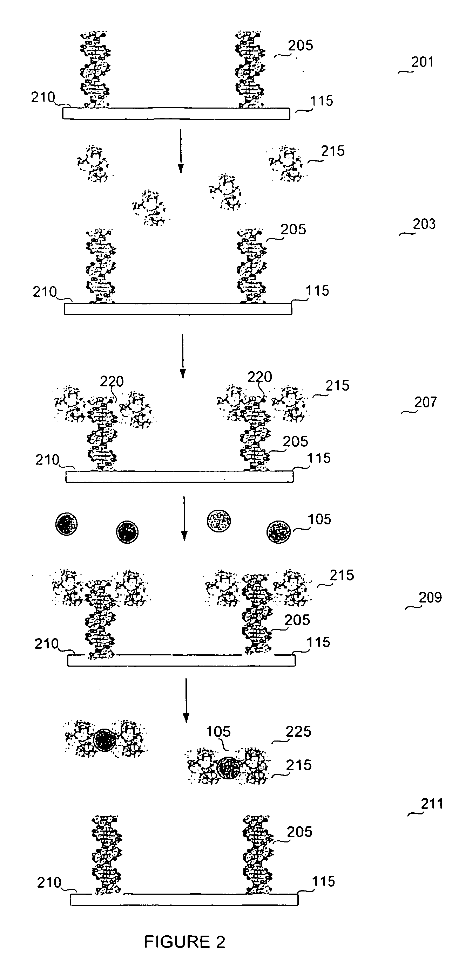

Figure 2 illustrates an expanded view of the CBS layer 115 and depicts mechanistic states for detecting the analyte 105 using the competitive binding approach to analysis. The CBS layer 115 comprises a plurality of substrate-bound molecules 205 that are fixed to the CBS layer surface 210 (state 201). The substrate-bound molecules may be fixed to the CBS layer surface 210 by covalent attachment or non-covalent attachment depending upon the surface chemistry of the CBS layer 115 and the reactivity of the substrate-bound molecules 205.

In the illustrated embodiment, the substrate-bound molecules 205 comprise nucleic acid strands or DNA molecules having a specific nucleotide sequence for which a plurality of release molecules 215 exhibit a reversible binding affinity. The release molecules 215 are desirably introduced into the environment where the substrate-bound molecules 205 are located (state 203) and allowed to bind along regions 220 of the substrate-bound molecules 205 where a first binding affinity is observed (state 207). In one aspect, the release molecules 215 comprise protein or peptide sequences, which exhibit a binding affinity for the specific nucleotide sequences within the substrate-bound molecules 205.

Although illustrated as double-stranded DNA molecules, it will be appreciated by those of skill in the art that other types of nucleic acid species may suitably used in conjunction with the biosensor device 100. For example, single-stranded DNA may be incorporated into the biosensor device 100 as well as RNA or other combinations of nucleic acid compositions. A single stranded DNA or RNA can form a hairpin-type helix, or a complex conformation of defined secondary and tertiary structure. Similarly, RNA-DNA hybrids can adopt complex conformations. Additionally, the release molecules 215 may comprise protein or peptide fragments as well as other molecules that exhibit a binding affinity for the substrate-bound molecules 205.

As noted above, a device of the invention can support the protein pre-bound to the nucleic acid, e.g., in a stable binding complex. In addition, the device can be supplied with the nucleic acid (or protein) immobilized, and reacted with the soluble binding partner just prior to contact with the sample for analyte detection.

Target analyte molecules 105 are subsequently introduced into the environment where the substrate-bound molecules 205 are bound to the release molecules 215 (state 209). The competitive release molecules 215 exhibit a second binding affinity for the analyte molecules 105 permitting uncoupling with the substrate-bound molecules 205 and binding to the analyte molecules 105. The binding of the release molecules 215 with the analyte molecules 105 forms a releasable complex 225 which may become dislocated and free to move into the environment surrounding the CBS layer 115 (state 211). This dislocation further renders the substrate-bound molecule 205 in an uncoupled state and is detected in a manner that will be described in greater detail hereinbelow.

The coupling of the release molecules 215 with the substrate-bound molecules 205 and the analyte 105 is determined by the identity of the dissociation factors and equilibrium binding rates which are dependent upon the concentrations of these molecules 215, 205, 105 with respect to one another. The dissociation factors and equilibrium binding rates further describe the proportion of release molecules 215 which are bound to the CBS layer 115 and may be used in optimizing the conditions and sensitivity of the analysis.

The identification and measurement of the concentration of the analyte 105 is desirably determined by assessing the state of coupling of the substrate-bound molecule 205 to release molecules 215. This manner of indirect analysis, by employing a larger signaling molecule (e.g., the protein) improves the sensitivity with which the target analyte 105 can be detected and permits the use of evanescent wave employing methods to rapidly accomplish the analysis. In one aspect, the interactions, which occur on the surface 210 of the CBS layer 115 are modeled after biological systems which incorporate a binding affinity for a target analyte 105. Suitable biological systems include the prokaryotic arsenic metabolism pathway from which both nucleic acid and protein sequences may be obtained that can be incorporated into the competitive binding approach to analysis described above. Furthermore, the sensitivity and conditions of the analysis can be altered by modifying the sequences of the nucleic acid and protein components, as necessary, to modify the dissociation factors and equilibrium binding rates between the analyte 105, the release molecules 215, and the substrate-bound molecules 205. For example, as exemplified, a DNA binding sequence comprising a dyad repeat can be halved to change binding affinity for the protein. The dynamic range of the biosensor device 100 may be desirably adjusted, as needed, by various combinations of the aforementioned modifications. In addition, by providing a mixture of binding sequences with different protein binding affinities, one can also increase the assay dynamic range. Such dynamic range adjustments are useful in not only increasing the sensitivity of the device to improve trace analysis, but also permitting the device to be used in applications where sample dilution is undesirable by reducing the sensitivity of the device.

In one aspect, the dynamic range of the biosensor device 100 is desirably between approximately 0.1 part per billion and 5000 parts per billion and more preferably between approximately 1 parts per billion and 3000 parts per billion and still more preferably between approximately 1 parts per billion and 1500 parts per billion. Furthermore, the biosensor device is capable of detecting target analyte 105 concentrations in the range of approximately 0.1 µg/ml and 5000µg/ml and more preferably between approximately 1 µg/ml and 3000 µg/ml and still more preferably between approximately 1 µg/ml and 1500 µg/ml.

The conditions for analysis using the biosensor device desirably comprise exposing the biosensor surface to the target analyte at a temperature between approximately 4°C and 45°C and more preferably between approximately 10°C and 40°C and still more preferably between approximately 18°C and 38°C. The time of analysis for which the biosensor device 100 should desirably be exposed to the target analyte 105 is between approximately 10 sec and 180 sec and more preferably between approximately 30 sec and 120 sec and time still more preferably between approximately 45 sec and 75 sec.

In one embodiment, the biosensor device can be fabricated using substrate-bound molecules 205 and release molecules 215 that are not necessarily naturally occurring biomolecules. One or both of these molecules 205, 215 can be synthetic biochemical molecules whose interaction is mediated by the target analyte 105. In another embodiment, substrate-bound molecules 205 and/or release molecules 215 that interact with the desired target analyte 105 can be identified by phage or ribosome display experiments. In still another embodiment, previously identified substrate-bound molecules 205 and/or release molecules 215 can further be used in other selection experiments to identify other suitable molecules that interact with one another or with the target analyte 105. These newly identified molecules can then be incorporated into a bioassay for the competitive detection of the presence of the target analyte 105.

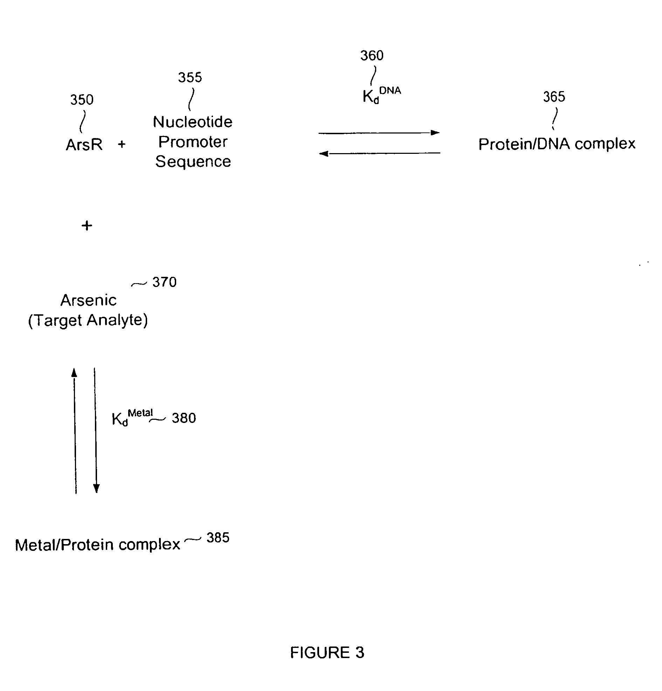

Figure 3 further illustrates the components of an exemplary prokaryotic arsenic metabolism pathway that may be used in conjunction with the biosensor device 100 to quantitate analyte molecules 105 comprising inorganic heavy metals. The biological molecules used in this system include components adapted from the *ars* operon for *Escherichia coli* used to confer resistance to oxidized ions of heavy metals (specifically arsenic). Other heavy metals ions detectable in this manner may include, for example, antimony, cadmium, chromium, lead, mercury, and tellurium among other molecules whose molecular interactions with components of biological molecules such as nucleic acids or proteins can be used for the purpose of detection and quantitation with the biosensor device 100.

In one aspect, the use of biological molecules and components that exist in biological pathways found in organisms such as *Escherichia coli* desirably provide a highly sensitive and specific means of detection with which target analyte molecules can be assayed. For example, as will be shown in greater detail hereinbelow the components of the *ars* operon are highly specific for particular inorganic ions such as arsenic and relatively insensitive to other inorganic ions such as phosphate, sulfate, nitrate, etc. Thus, the incorporation of these biological molecules and pathways into the CBS layer 115 allows the biosensor device 100 to selectively detect and quantitate the analyte molecules 105 while remaining relatively insensitive to other structurally or chemically similar molecules which might otherwise lead to false quantitation or detection of the target analyte 105 of interest should they be present in an analyte sample.

In using the *ars* operon, a protein (ArsR) is produced by the gene encoding for one component of arsenic resistance in Escherichia coli and other prokaryotic as well as some eukaryotic organisms. The ArsR protein possesses highly selective arsenic binding properties while being relatively insensitive to other non-metal ions. Furthermore, the ArsR protein exhibits properties of DNA binding affinity to a nucleotide sequence comprising the transcription promoter region of the *ars* operon. Thus the ArsR protein and companion transcription promoter region nucleotide sequence can be used to form the competitive binding components necessary to detect arsenic molecules using the biosensor device 100.

As shown in Figure 3 the ArsR protein 350 exhibits a characteristic binding affinity for the nucleotide promoter sequence 355 which can be measured as a function of equilibrium dissociation constant (K_{d}^{DNA}) 360. As binding between the ArsR protein 350 and the nucleotide promoter sequence 355 occurs a protein/DNA complex 365 is formed comprising the ArsR protein 350 non-covalently coupled to the nucleotide promoter sequence 355. Incorporating these molecules into the biosensor device 100 can be accomplished where the nucleotide promoter sequence 355 serves as the substrate bound molecule 205 and the ArsR protein 350 serves as the release molecule 215.

Arsenic metal molecules 370 (representing the target analyte 105 in Figure 2) further posses a binding affinity for the ArsR protein 350 and form a metal/protein complex 385 with a characteristic dissociation constant (K_{d}^{metal}) 380. Thus, as arsenic molecules 370 are introduced into the region where substrate-bound molecules 205 (comprising the nucleotide promoter sequence 355) are present, a competitive binding occurs with respect to the ArsR protein 350. As arsenic molecules 370 bind the ArsR protein 350, the amount of nucleotide promoter sequence molecules 355 which remain in the protein/DNA complex form 365 is reduced. This reduction in the protein/DNA complex 365 is observable as a change in reflected light properties when coupled to an evanescent wave-employing device. Further details of these interactions and how they are used for analyte quantitation with be provided in subsequent figures.

Further explanation of the *ars* operon and determination of dissociation and equilibrium constants can be found in Environmental Chemistry of Arsenic; Simon Silver, et al.; Chapter 9, Marcel Dekker Publishers (2001) and "Determination of Rate and Equilibrium Binding Constants for Macromolecular Interactions by Surface Plasmon Resonance"; Daniel J. O'Shannessy, et al.; Methods of Enzymology; Vol 240; Academic Press Inc. (1975). It must be understood however that for any given system, thermodynamic binding analysis from the literature may be inaccurate or incomplete, particularly for a given system under study. Thus, dissociation and equilibrium constants from the literature may or may not take account of pH, redox, and salt conditions present in a given system or found in a given sample. For this reason, empirical testing and adaptation on an assay system or device contemplate for use may be required to arrive at satisfactory or optimum conditions with respect to these variables.

Additionally, details of evanescent wave detection methods can be found in "Biospecific interaction analysis using biosensor technology"; Magnus Malmqvist; Nature; Vol 361; (1993).

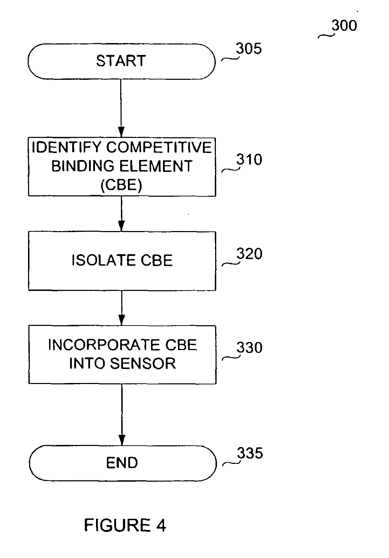

Figure 4 illustrates a method 300 for the initial preparation of the biosensor device 100. The process 300 begins in a start state 305 and proceeds to a state 310 where competitive binding elements (CBEs) corresponding to the substrate-bound molecules 115 which are suitable for biosensor 100 incorporation are identified 310. In this state 310, nucleic acid and/or peptide sequences are identified which possess binding affinity for the desired analytes 105. Desirable characteristics of the nucleic acid and peptide sequences include the ability to be bound to the analyte 105 with a high degree of specificity and furthermore at relatively low concentrations. Such binding may be reversible or irreversible in nature and may occur over a variety of different conditions (i.e. temperature, concentration, physical state). The specificity of the nucleic acid and peptide sequences binding affinity is important to insure that the biosensor 100 registers only desired analytes 105 of interest while minimizing false signals or high background from the experimental mixture 107 for which the analysis is performed.

As previously described one exemplary category of biosensor compatible molecules comprises the prokaryotic resistance gene cassette or operon comprising the genes encoding for the metabolism of arsenic in bacteria which may be used as a model for preparing a suitable biosensor 100. In this system, genes associated with the metabolism of arsenic code for proteins that have a multifunctional affinity for both promoter element nucleic acid sequences and arsenic molecules. These proteins may be used in conjunction with the portions of the nucleic acid sequence coding for the promoter element to provide the necessary substrate-bound 205 and competitive release molecules 210 used in the detection of the arsenic analyte 105.

It will be appreciated by those of skill in the art that numerous biological pathways and molecules exist whose components may be used in a similar manner to that described in the aforementioned description of the biosensor device 100. It is therefore conceived that use of other biological pathways and molecules in conjunction with the biosensor device 100 in the manner described herein represent but other embodiments of the present invention.

Following identification of the CBEs in state 310, the process 300 proceeds to a state 320 where the CBEs are isolated. In this state 320, nucleic acid sequences corresponding to the substrate-bound molecules 205 may be incorporated into a suitable plasmid vector and expanded by propagation in a host cell or amplified by polymerase chain reaction to produce a sufficient quantity of the substrate-bound molecules to coat the surface 210 of the competitive binding layer 115 of the biosensor 100. Additionally, the protein or peptide sequences corresponding to the competitive release molecules 215 may be synthesized directly or obtained by appropriate translational methods.

The isolated CBEs are subsequently incorporated in the sensor 100 in a state 320 to prepare the competitive binding surface layer 115. As previously indicated, based on the dissociation factors of the CBEs, a desirable seeding density for each CBE may be determined which desirably increases the sensitivity of the biosensor. Furthermore the isolated CBEs may be modified to alter their affinity for the analyte 105 to produce desirable equilibrium and dissociation properties between the components of the biosensor surface. Following CBE incorporation 330, the process 300 then proceeds to a terminal state 335 to complete the initial preparation of the biosensor surface layer 115.

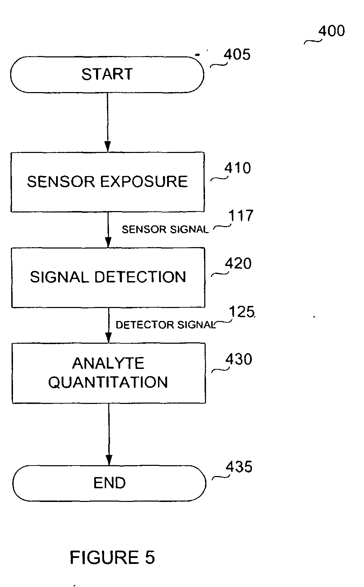

Figure 5 illustrates a method 400 for qualitative and quantitative assessment of the presence of the analyte 105 in the experimental mixture 107. The method 400 begins in a start state 405 and proceeds to a state 410 wherein the sensor 100 is positioned in proximity to the experimental mixture 107 such that the CBS layer 115 is exposed to the analyte 105. As will be described in greater detail in subsequent illustrations, the analyte 105 selectively binds to the release molecules 215 resulting in their uncoupling from the substrate-bound molecules 205. The rate and degree of uncoupling is in proportion to the amount of analyte 105 present in the experimental mixture 107 and results in the production of the aforementioned sensor signal 117.

The sensor signal 117 is detected in a new state 420 where the sensor signal 117 is electronically transformed or amplified to produce the detector signal 125 which is subsequently used in the quantitation of the analyte in a state 430. The quantitation state 430 determines the amount of analyte 105 in the experimental mixture 107 based upon the detector signal 125 using a calibrated signal intensity reference which correlates the sensor signal intensity with corresponding concentrations of analyte 105. Upon quantitating the analyte 105, the method 400 stores the results in an electronically accessible form, which may be output to the user and the process proceeds to an end, state 435.

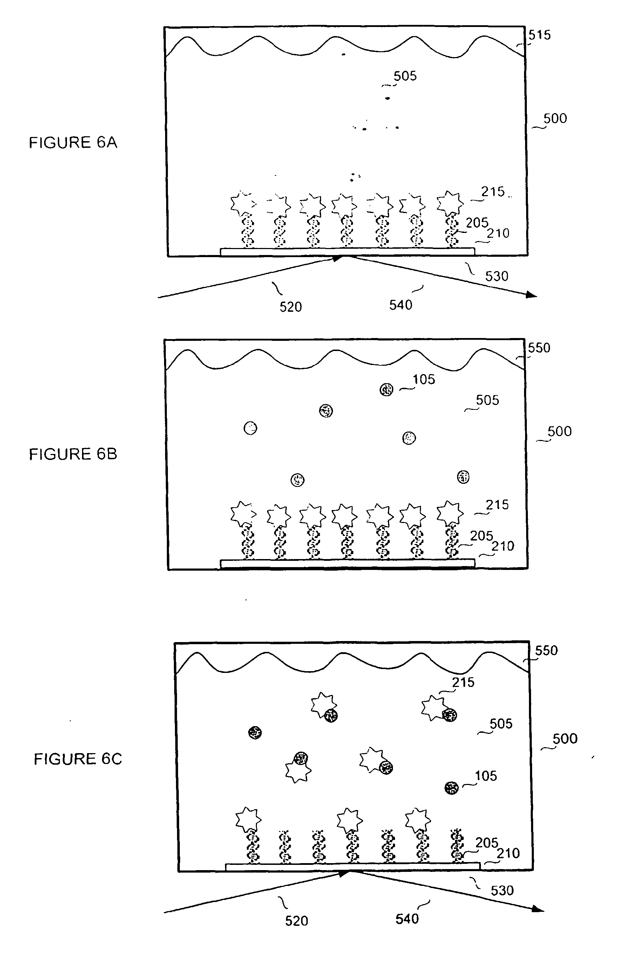

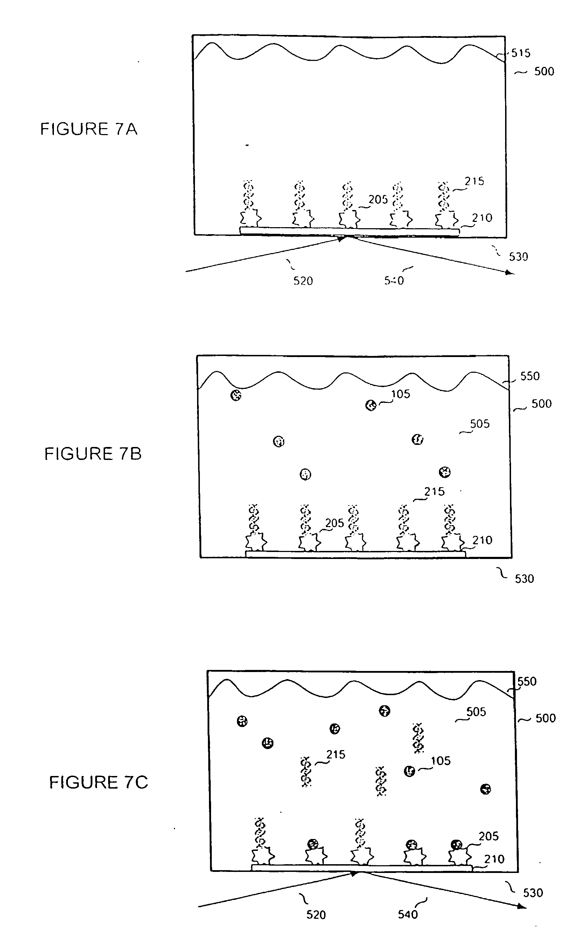

Figures 6 and 7 further illustrate sensor schematics for the analysis apparatus 500 used to detect and measure the concentration of the analyte 105. In Figures 6A-C, and 7A-C two modes of binding are shown in which the release molecules 215 possess selective affinity for the analyte 105. As shown in Figures 6A the CBS layer 115 comprises the substrate-bound molecules 205 fixed to the upper surface 210 and further comprises release molecules 215 occupying affinity sites on the substrate-bound molecules 205. When a solution or mixture 515 is introduced into an exposure region 505 of the analysis apparatus 500, a characteristic reflectivity can be measured by directing light 520 towards the lower surface 530 of the CBS layer 115. The intensity of the reflected light 540 is then measured as previously described to obtain a baseline measurement of the reflectivity of the solution or mixture 515.

Subsequently, a solution or mixture 550 containing the analyte 105 of interest is introduced into the exposure region 505 (Figure 6B) and results in competitive binding of the analyte 105 to the release molecules 215 (Figure 6C). The aforementioned binding liberates of at least a portion of the release molecules 215 into the solution or mixture 550 leaving at least a portion of the substrate-bound molecules 205 with free or unoccupied affinity sites. Redirection of light 520 against the lower surface 530 of the CBS layer 115 is registered as a change in intensity of the reflected light 540 resulting from the change in binding state of the substrate-bound molecules 205. The change in intensity between the two states of affinity site occupation of the substrate-bound molecules 205 therefore can be compared and calibrated to determine the concentration of analyte 105 in the solution or mixture 550.

It will be appreciated that the aforementioned analysis apparatus 500 may be used to measure analyte 105 concentrations in a number of different solutions or mixtures including those with differing elemental states including solid, liquid, and gaseous states. Furthermore, the analysis apparatus 500 may incorporate a plurality of different substrate-bound molecules 205 with varying affinities for different target analytes 105. Thus the analysis apparatus 500 may be used to measure more than one type of target analyte 105 increasing its flexibility and functionality.

An additional feature of the analysis apparatus 500 is that it can be fashioned in such a manner so as to be reusable or rechargeable to permit multiple analyses to be sequentially performed. For example, after conducting a first analysis with the apparatus 500, the CBS layer 115 can be washed and a solution containing only the release molecules 215 (lacking any analyte 105) allowed to bind to the previously unoccupied binding sites of the substrate-bound molecules 205. This restores the CBS layer 115 to the initial state where the apparatus 500 is ready to again receive target analyte molecules 105 for the purposes of quantitation and analysis. Furthermore denaturing agents including, but not limited to, urea, guanidine hydrochloride, and sodium dodecyl sulphate may be used to partially or wholly restore the target analyte 105 binding capacity of the biosensor device 100.

Figures 7A-C illustrates another embodiment of an analysis apparatus 500, wherein the substrate-bound molecules 205 possess a competitive binding affinity for both the release molecules 215 and the target analyte 105. In this apparatus 500 the CBS layer 115 is prepared, as before, by reversibly binding the release molecules 215 to the affinity receptors on the substrate bound molecules 205 (Figure 6A). As analyte 105 is introduced into the exposure region 505 (Figure 6B), the analyte 105 binds directly to the substrate bound molecules 205 with a concomitant release of the release molecules 215 (Figure 6C). These alterations in binding of the substrate bound molecules 205 can be detected as before by measuring changes in the intensity of light 520 reflected off of the lower surface 530 of the CBS layer 115.

It will be appreciated that the aforementioned apparatus 500 may desirably employ a peptide or protein molecule with selective binding affinities for both a specific nucleotide sequence and a target analyte molecule such as arsenic. In this embodiment, the arsenic molecules bind directly to the peptide or protein molecules affixed to the CBS layer 115 resulting in the release of the nucleotide molecules. The state of binding of the molecules affixed to the CBS layer 115 is then measured as a function of the change in intensity of the reflected light 540.

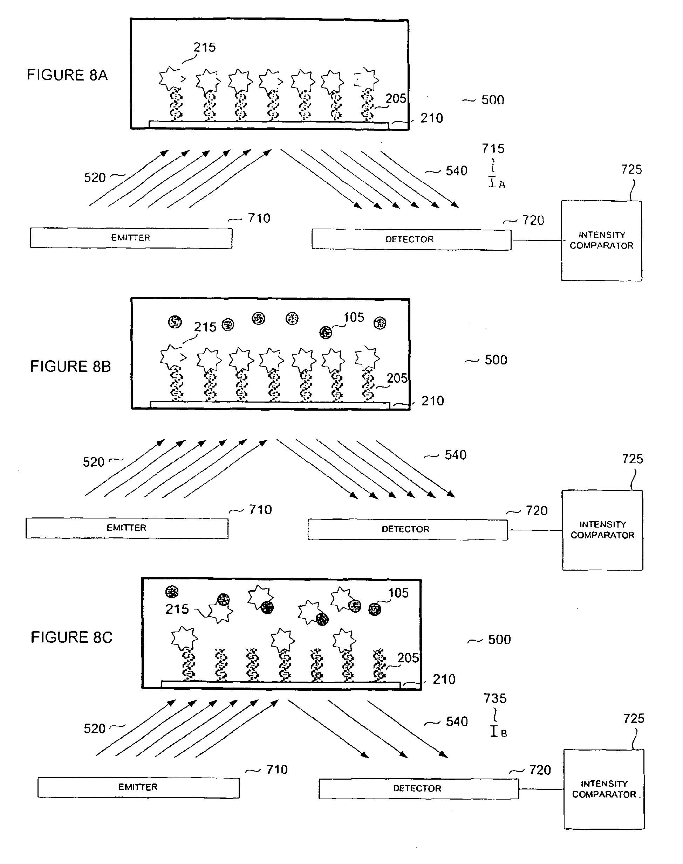

Figure 8A-C illustrate another embodiment of the biosensor device 500 wherein a light emitting source 710 is positioned to emit light 520 in the direction of the CBS 115. The light source 710 may comprise any of a number of different light producing elements including incandescent, laser, and LED emitting light sources. Furthermore, the light may be polarized or filtered to produce wavelength-specific light which desirably improves the quality of illumination and reflection on the CBS layer 115. As shown in Figure 8A, and as previously described, when the sensor device 500 is not exposed to the target analyte 105 the emitted light 520 is reflected with a first sampling intensity (I_{A}) 715. The reflected light 540 is then projected against a detector 720 capable of measuring the reflected light 540 and quantitating the first sampling intensity 715. The detector 720 comprises a photo-detector or other photosensitive element capable of detecting and quantitating small differences in the change of intensity of light to which it is exposed. An intensity comparator 725 receives signals from the detector 720 indicative of the reflected light intensity and processes this information to determine the concentration of the target analyte 105 in a manner that will be described in greater detail hereinbelow.

As the analyte 105 is exposed to the sensor surface 210 (Figure 8B) a change in the intensity of reflected light 540 may be observed resulting in the production of a second sampling intensity (I_{B}) 735 (Figure 8C). The second sampling intensity 735 is recorded by the detector 720 indicating the relative amount of analyte 105 bound to the appropriate molecules in the sensor 500. Quantitation of the analyte 105 proceeds as the intensity comparator 735 compares the first sampling intensity 715 (analyte absent) and the second sampling intensity 735 (analyte present) to generate a value representative of the concentration of the analyte 105. In one aspect, a standard curve or calibration table may be used by the intensity comparator 725 to determine the concentration of the analyte 105. The standard curve associates known concentrations of the analyte 105 with expected light intensities to provide a rapid means to identify the concentration of the analyte 105.

One feature of the biosensor device 500 is that it may be configured to operate in a real-time acquisition mode wherein a plurality of intensity measurements are recorded over a period of time to monitor the concentration of analyte 105 in a sequential manner. Thus, a gaseous or liquid sample may be passed over the biosensor surface 210 and changes in the concentration of the analyte 105 may be detected and quantitated in a continuous manner by sampling the reflected light intensity at various times. This feature of the biosensor device 500 improves its flexibility and allows the biosensor device 500 to be used in applications where a plurality of concentration or quantitation measurements of the analyte 105 are to be made without replacing or exchanging the sensor 500.

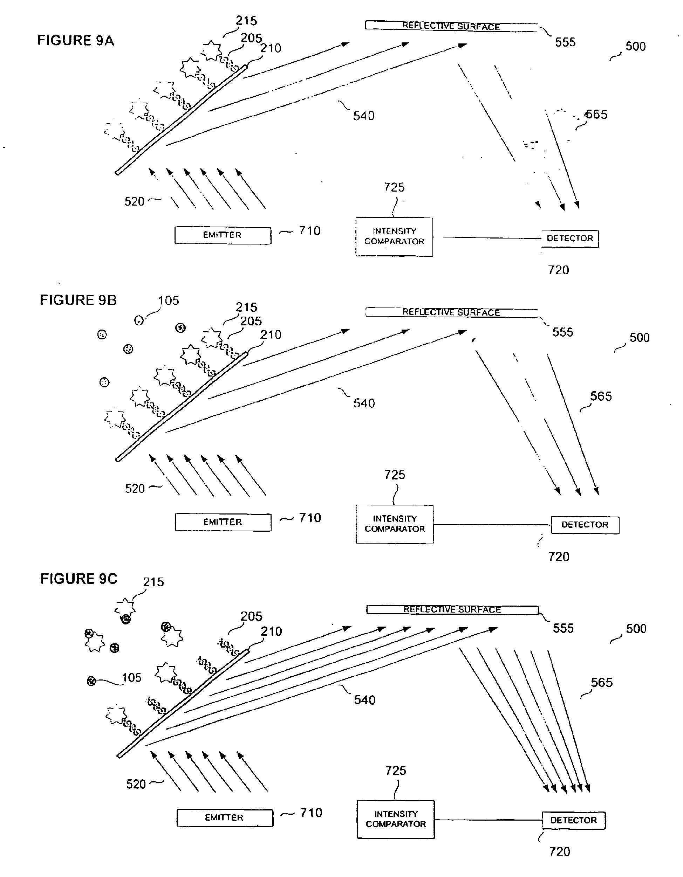

Figure 9A-C illustrates another embodiment of the biosensor device 500 incorporating a secondary reflective surface 555 able to redirect reflected light 540 from the sensor surface 210 at an angle which can be subsequently captured by the detector 720. In one aspect the addition of the secondary reflective surface 555 increases the flexibility in positioning the components of the biosensor device 500 and improves the capture and quantitation of the reflected light 540. The reflective surface 555 may further serve as a focusing surface which receives a relatively dispersed quantity of reflected light 540 from the sensor surface 210 and subsequently narrowly transmits the light in a new direction such that the redirected light 565 may be focused on a small receiving region of the detector 720. Thus, the incorporation of the reflective surface 555 facilitates the construction of biosensor devices 500 of reduced size and enables detectors with relatively small detection surfaces to be used.

As previously indicated the biosensor device 500 may be used to detect a number of analyte molecules based on competitive coupling and release properties. In one aspect, biological molecules such as nucleotide strands and proteins may be used to selectively and competitively bind to the analyte 105. These molecules are desirably fixed to the biosensor surface 210 as described above to produce the detection signal used for quantitation of the analyte 105.

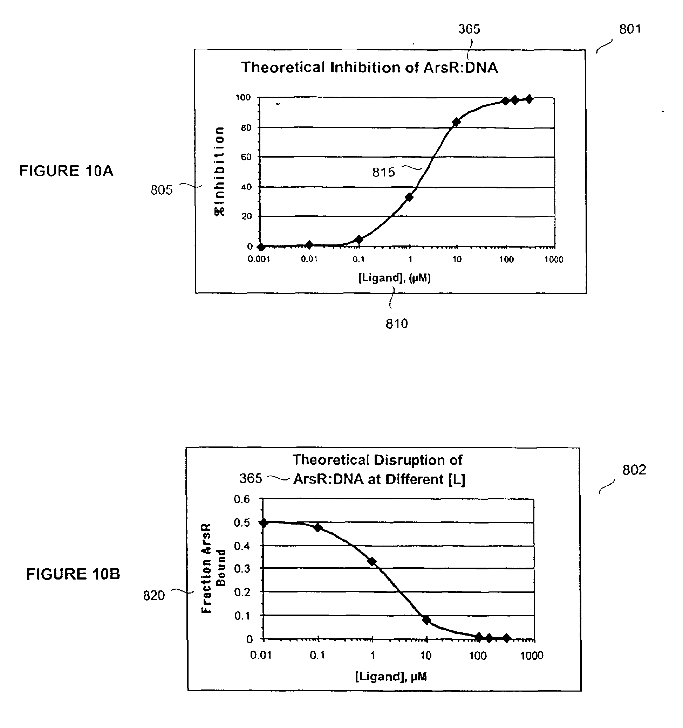

Figure 10A-B illustrate graphs 801, 802 which demonstrate the suitability and sensitivity of competitive binding mechanism as applied to the ArsR protein, Ars operon, and arsenic ligand. In Figure 10A, an inhibition graph 801 depicts the relative coupling of the ArsR/DNA complex 365 measured as a function of percentage inhibition 805 in ArsR/DNA complex formation based on the presence of various concentrations of arsenic molecules 810. An inhibition curve 815 further demonstrates that increasing the concentration of ligand arsenic 810 results in an increase in the inhibition of ArsR/DNA complex formation. The inhibition graph 801 is useful in identifying the sensitivity range and absolute limits of detection of the competitive binding system 100.

In one aspect, the components of the competitive binding system 100 may be manipulated or modified, for example by sequence alteration, to obtain system components with greater dynamic range and accuracy. By graphing the results of modified components and concentrations of the competitive binding system 100, the sensitivity of the system 100 may be determined. Furthermore, the inhibition graph 801 may be used to determine the appropriate amounts of substrate-bound molecules and release molecules to be used with the evanescent wave employing device to achieve a desirable reflectivity signal for the purpose of analyte detection and quantitation.

Figure 10B illustrates another competitive binding graph 802 wherein the ArsR/DNA complex 365 is measured as a function of ArsR fraction bound 820 at various concentrations of ligand arsenic 810. As described previously, information obtained from this competitive binding graph 802 is useful in analyzing the components and efficiency of the system. Furthermore, this information may be used to develop appropriate CBS layer compositions to be used in the biosensor device 100.

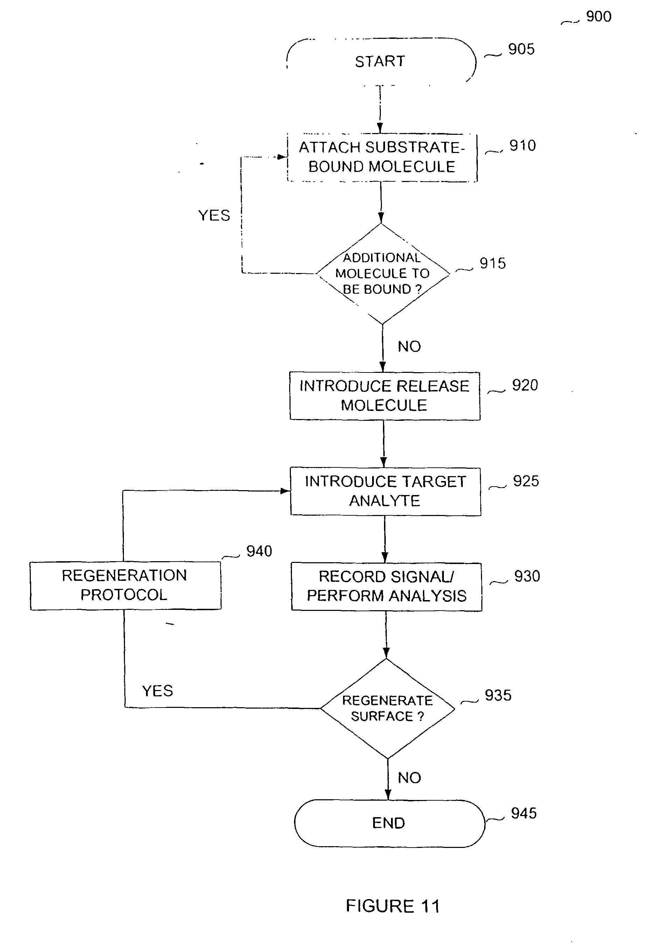

Figure 11 illustrates a process 900 for preparation and analysis using the aforementioned biosensor device 100. The process begins in a start state 905 and proceeds to a state 910 where substrate bound molecules 205 are attached to the sensor surface 210. As previously indicated, more than one type of molecule may be bound to the sensor surface 210, for example to provide detection of multiple target analyte types 105. The determination to bind multiple types of substrate bound molecules 205 is handled in a decision state 915 which proceeds to a state 920 where the release molecules 215 are introduced. In this state, the release molecules attach to the substrate-bound molecules to prepare the sensor 100 for analysis of the target analyte 105.

In state 925, the target analyte 105 is introduced and exposed to the sensor surface 210 to produce a discernable signal which is recorded in state 930. During the analysis state 930, a signal difference analysis may be performed that compares the signal produced by the presence of the target analyte 105 relative to the signal generated when only release molecules 215 are bound to the substrate-bound molecules 205 in the absence of target analyte 105.

In one aspect, standard curves may be constructed and used to aid in the determination of the concentration of the target analyte 105 based on the measured signal. These standard curves may further be prepared using solutions with known concentrations of target analyte 105 or inhibitors to the binding of the target analyte 105 that aid in the determination of the analyte concentration in a test solution.

Following target analyte detection and quantitation in state 930, the process 900 proceeds to a state 935 where a decision may be made to regenerate the detection surface 210 of the biosensor device 100. If regeneration is desired, a regeneration protocol is used in state 940 to prepare the sensor 100 for reintroduction of the target analyte 105 in state 925. If regeneration is not desired, the process 900 reaches an end state 945 completing the preparation and analysis process 900.

It will be recognized that although the biosensor device 100 has been described for use in an inorganic molecule detection system, this device 100 can be readily adapted for use in detecting and quantitating numerous other types and varieties of molecules (analytes). These analytes may include organic and inorganic molecules and ions. Furthermore, the size and molecular weight of the target analyte 105 is not limiting and thus the device 100 can be configured to detect both large and small molecules alike.

Based, in part, on the sensitivity of the biosensor device 100 and the specificity and sensitivity of the substrate-bound molecules 205 and release molecules 215 used in the device 100, the biosensor may have a sensitivity or detectability threshold in the picomolar or even attomolar range. Additionally, the size of the molecules that can be detected may have a molecular weight less than 1000 and more preferably less than 500 and still most preferably less than 150.

### Small Molecule Targets for Biosensors of the Invention

In addition to the metal compounds described above, some specific examples of molecules that can be detected by various configurations of the biosensor device 100 include: methyl tertiary-butyl ether (MTBE), a commonly used gasoline oxygenate additive; N-nitrosodimethylamine (NDMA), used in rocket fuel production, as plasticizer for rubber, in construction of batteries, and in the synthesis of polymers and co-polymers; and 1,4-dioxane, used as a solvent stabilizer in organic chemical manufacturing, and as a wetting and dispersing agent in textile processing.

### Small Molecule Binding Proteins

As noted above, various possibilities are available for proteins that bind to small molecule targets and to specific DNA sequences, but not to both simultaneously. Generally, proteins are selected on the basis of specificity of binding to both DNA and the small molecule target. Moreover, through routine molecular biological techniques, it is possible to alter the DNA or small molecule binding characteristics of the protein to increase the dynamic range of the system, increase specificity, and increase sensitivity. The effects of the modifications set out below can be determined through routine binding assays, both kinetic and concentration dependent, or combinations thereof.

Clearly naturally occurring metal-response operons provide a good source for such proteins, and the DNA molecules to which they specifically bind (see, e.g., various World Wide Web sites; US 5,571,722). For example, a first alternative embodiment of the invention may involve MerR (a repressor protein for the mercury resistance operon) in combination with the Mer operon. As Hg(II) ions attach to the repressor protein MerR, MerR behaves differentially with respect to the binding site on the promoter, and consequently with respect to that binding site on an oligonucleotide.

Similarly, a second alternative embodiment of the invention may involve CadR and the Cadmium operon. The cad operon contains a cadC gene whose product negatively regulates transcription. The addition of Cd(II) ions relieves the repression, by removing the repressor protein CadR from the promoter. It has been shown that CadR also binds zinc and lead.

A third alternative embodiment of the invention may involve smtB. smtB is a metal-dependent (specifically Zn(II) ions) repressor of the cyanobacterial metallothionein gene smtA. As such, the presence of Zn(II) ions mediate a protein-DNA complex.

In a further embodiment, naturally occurring proteins are modified to alter binding characteristics. For example, it a specific embodiment amino acids 98-117 are deleted from the ArsR protein shown in SEQ ID NO:2, yielding a protein corresponding to amino acid residues 1-97 of SEQ ID NO:2. Alternatively, removal of the C-terminal cysteine (amino acid 116) might stabilize the protein. Similar modifications are possible on any of the small molecule-DNA binding proteins for use in practicing this aspect of the invention. Such a protein fragment may also be fused with another protein, such as a purification handle (like hexahistidine, FLAG, GST, etc.). In a specific aspect, the protein comprises a sequence of a bacterial DNA-binding regulatory protein encoded by a metal-response operon sufficient to bind DNA and the metal. For example, an ArsR protein may have an amino acid sequence that is at least 90% identical to the amino acid sequence of amino acids 1-97 of SEQ ID NO:2, which ArsR protein binds to a nucleic acid sequence selected from the group consisting of SEQ ID NOS: 3, 4, 5, 6, 7, 8, 9, and 10.

Alternatively, introducing amino acid substitutions into the small molecule binding protein can alter the DNA binding properties or small molecule binding properties, or both, as needed.

In addition, known proteins, particularly the known metal binding proteins, can be modified through the techniques of directed evolution and gene shuffling described above to generate proteins with specificity for different metal ions or metal ion complexes, and to generate proteins with different small molecule binding specificity.

Regardless of the source of the protein or gene encoding it, one will employ routine molecular biological techniques to express high levels of the protein. Thus, in accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription And Translation [B.D. Hames & S.J. Higgins, eds. (1984)]; Animal Cell Culture [R.I. Freshney, ed. (1986)]; Immobilized Cells And Enzymes [IRL Press, (1986)]; B. Perbal, A Practical Guide To Molecular Cloning (1984); F.M. Ausubel et al. (eds.), Current Protocols in Molecular Biology, John Wiley & Sons, Inc. (1994).

### Nucleic Acids

Various nucleic acids are known to contain specific binding sequences for proteins that bind to the DNA and release when bound by a small molecule (the repressor systems described above are well known examples of this behavior). Preferably, the DNAs contain linkers if they are to be attached to the solid phase support. One exemplary linker is biotin. Biotin-labeled nucleic acids will adhere to avidin or streptavidin coated surfaces. In a specific embodiment, e.g., for use with a Spreeta^{™} device surface, the binding molecule is neutravidin. Other linkers are well known for binding DNA to microarray supports. Direct attachment of the nucleic acid to the support is also envisioned. Furthermore, the oligos that form the double stranded DNA can be of asymmetric length, again to enhance linker binding to the solid support. Immobilizaton of nucleic acids is well-known (see, e.g., Hermanson, Bioconjugate Techniques, Academic Press: Boston, 1996).

Furthermore, as with the proteins, modifications of the nucleic acid sequences provide for altering the protein binding interaction. The nucleic acid may be modified to contain a sequence that differs by at least one nucleotide from a specific binding sequence from promoter or other regulatory protein binding sequence, and which nucleic acid is specifically bound by the protein with different binding constants than the unmodified sequence. For example, the invention envisions an oligonucleotide having a sequence that differs by no more than three bases or base pairs from a sequence selected from the group consisting of SEQ ID NOS: 3, 4, 5, 6, 7, 8, 9, and 10. Generally, at most the number of nucleotides that will be modified will not exceed 10%, or three or fewer base changes in a 30-mer oligonucleotide. Furthermore, as demonstrated in the examples, it is also possible to halve a dyad repeat, resulting in a shorter DNA with lower affinity binding (which may be desirable for the particular system involved). In still another embodiment, in which DNA is immobilized, a combination of different molecules with different sequences is immobilized to provide a heterogeneous assortment of binding characteristics. This heterogeneous assortment will not adversely affect the signal output, but could significantly enhance the dynamic range of an assay. As with the protein modifications, it is routine to conduct binding assays to determine whether a particular modification has a desired effect.

### Detection Systems

Numerous detection systems are available for detecting binding or absence of binding of the first class of molecules (e.g., protein) to the second class of molecules (e.g., nucleic acid). Furthermore, quantitation of binding can be achieved through absolute values of measure interaction, or in some cases more accurately by measuring the kinetics of binding or release. For kinetic-based analyte detection, the detection system is preferably connected to a microprocessor device, e.g., a PC board, for data acquisition and analysis. Thus, a device of the invention preferably provides a determined value of analyte concentration, rather than raw data for independent calculation of that value.

Surface plasmon resonance is a method well known in the art. Preferably, the concentration of the analyte is determined by assessing the state of coupling of the substrate-bound molecules to the release molecules. Preferably, the property of the substrate-bound molecule, which undergoes a measurable change, is an optical property. Though the evanescent wave employing methods are the preferred embodiment, one skilled in the art will appreciate that other sensor methods can be employed in conjunction with the specific biological recognition mechanism as disclosed herein. Those skilled in the art will appreciate the technology that can compose the detector mechanism of the biosensor. These include any electrochemical transducer, including potentiometric, amperometric, or optical-based transducer mechanisms, including fiber optic systems employing different wavelengths for analysis of analyte capture (e.g., infrared light). Many of these methods are known and disclosed in the art (see, e.g., U.S. Patent Nos. 6,063,573; 6,087,100; 6,071,699; 5,952,172; and 5,591,578). WO 89/05977 discloses commonly employed electrochemical and optical transducers incorporated in a biosensor. Preferably, the apparatus is associated with the relevant instruments and computer control and data-processing means for performing assays. Suitable instrumentation, computer control and data-processing means are well known.

It is conceived that the aforementioned biosensor fabrication and utilization methods may be adapted to operate in conjunction with numerous other biosensor devices including those using colorimetric or fluorescent-based detection methodologies. As an example of a fluorescent-based detection methodology using the competitive binding aspects of the biosensor system, the identification and quantitation of biochemical molecules can be detected using (Forster) resonance energy transfer (FRET) or fluorescence quenching. These solution-based methods do not require SPR for detection of the target analyte and may be used in conjunction with the competitive-binding approach to target analyte identification and quantitation to provide a highly sensitive means of detecting biomolecules.

Briefly described, FRET-based methods of detection include labeling one of the competitive binding molecules with a fluorescence donor and the other competitive binding molecules with a fluorescence acceptor such that the emission wavelength of the donor and the excitation wavelength of the acceptor are coincidental. Using a fluorescence spectrophotometer, or similar detection means, quantification of the interaction between these molecules is possible. The molecules used in these fluorescence-based detection systems may further possess natural or induced fluorescence that changes in wavelength or intensity upon interaction of the target analyte 105 and thus used to quantify the presence of the analyte. Using this biosensor configuration, the measurement of the interaction(s) between the various fluorescent molecules is attributable to the presence of the target analyte of interest and thus may be used for the purposes of identification and quantitation.

WO 02/10750 discloses fluorescence polarization where a fluorescently labeled analyte is bound to a biopolymer. The reference defines a biopolymer as including a protein. When analyte is added in a sample, it displaces the labeled analyte from the protein. The liberated fluorophone has a significantly lower polarization than the bound fluorophone, resulting in a detectable change in the signal. A fluorescent wavelength shift is based on the finding that many fluorophores exhibit a change in excitation and/or emission wavelength and quantum yield of fluorescence when released from a site on the biopolymer to an aqueous environment.

The reference further discloses fluorescence quenching where a change in fluorescence is dependent on the binding of an analyte to a biopolymer and the fluorophore is designed to be quenched, as well discloses a detection method involving the use of colorimetric quenching. An unstable dye molecule is bound to the biopolymer. When bound to the biopolymer, the dye is unreactive. However, upon analyte binding, the dye is displaced and becomes reactive.

Fluorescence spectroscopy is a technique well suited for very small concentrations of analyte. Fluorescence provides significant signal amplification, since a single fluorophore can absorb and emit many photons, leading to strong signals even at very low concentrations. In addition, the fluorescence time-scale is fast enough to allow real-time monitoring of concentration fluctuations. The fluorescent properties only respond to changes related to the fluorophore, and therefore can be highly selective. Many fluorescent chemosensors, including fluorophore-labeled organic chelators and peptides have been developed for metal ion detection. WO 02/00006 discloses a preferred embodiment of the biosensor comprising a fluorophore and a quencher arranged in proximity such that prior to cleavage the fluorescence intensity is decreased by the quencher. However, upon cleavage, the fluorophore and quencher are separated leading to an increase in fluorescence intensity.

Methods for detecting fluorescence are well developed. WO 99/27351 describes a monolithic bioelectronical device comprising a bioreporter and an optical application specific integrated circuit. The device allows for remote sampling for the presence of substance in solution. Furthermore, fluorometers for uses in the field are commercially available. Fluorescent detection is compatible with fiber optic technology. Several fluorescence-related parameters can be assessed for the purpose of sensing, including fluorescence intensity, emission or excitation wavelength, fluorescence lifetime and anisotropy. Fiber optic detection method is disclosed in Ewing, K.J., Nau, G., Jaganathan, J., Bilodeau, T., Schneider, I., Aggarwal, I.D., Fiber Optic Flow Injection Sensor for Determination of Heavy Metals in Water, Naval Research Laboratory, Optical Sciences Division, Code 6503.2, Washington, D.C. 20375.

U.S. Patent No. 5,459,040 discloses a sandwich assay, which relies on the ability of an analyte to form an aggregate or complex with two complexing chelators. One of the complexing agents is immobilized on a solid support and is capable of binding with the metal ion, while the other complexing agent is linked or bound to a reporter group and is capable of binding with the chelate complexed to form a sandwich chelate complex. The reporter group refers to any moiety, molecule, atom or species that results in a detectable signal or change when the analyte of interest is present in the sample. The reporter group includes enzymes in combination with an indicator, chromogens, fluorophores, radioisotopes, and biotin, or these groups attached to an antibody that recognizes the sandwich chelate. Specifically, the reporter groups maybe capable of catalyzing the formation of a fluorescent signal, a phosphorescent signal, a bioluminescent signal, a chemiluminescent signal, or an eletrochemical signal. Therefore, the presence of the sandwich chelate complex is detected by the presence or absence of the reporter group.

The reference also discloses a competitive method based on the use of an organometallic compound which competes with the metal ions present in the sample for the chelator. In this method, a chelator is immobilized on a solid support that is capable of binding with the metal ion to form a chelate complex. An organometallic compound immobilized on a reporter group, that is capable of binding to the metal ion to form a chelate complex, is added to the sample. The presence of the metal ion is detected through the presence or absence of the reporter group.

The use of a force amplified biological sensor (FABS) is disclosed in Baselt, D.R., Lee, G.U., Colton, R.J., A Biosensor based on Force Microscope Technology, Naval Research Laboratory, Code 6177, Washington, DC 20375-5342 and can be used to detect such reporter groups. The FABS is based on an immunobead sandwich assay that uses a cantilever to detect captured immunobeads. The number of magnetic beads present is proportional to the concentration of analyte in the sample.

Electrochemical detection systems further include potentiometric (e.g., pH, selective ion level measurement) and conductive changes (i.e. changes in resistance). Such methods include the use of biosensor biopolymers, including proteins, that upon binding of an analyte produce an electrochemically detectable signal, an amperometrically detectable signal, a potentiometrically detectable signal, a signal detectable as a change in pH, a signal based on specific ion levels, a signal based on changes in conductivity, a piezoelectric signal, a change in resonance frequency, a signal detectable as surface acoustic waves, a signal detectable by quartz crystal microbalances, or the like.

In addition to detecting various events associated with the state of coupling of the substrate-bound molecule to the release molecules, these transducers is also capable of generating information associated with those events by transducing or converting the events into a measurable quantity such as an electronic or optical signal. These signals are then channeled via connections well known in the art to a device which processes the information represented into displays, records, digitized data, etc. The particular device depends on the type of physical or chemical phenomenon being detected. Electrochemical transducers are often coupled with electrometers, voltmeters, impedance analyzers, and capacitance bridges. Photomultiplier tubes, photodiodes, phototransistors, spectrophotometers, monochromators, and photon counters are among the devices commonly used to process information generated by optical transducers.

While the aforementioned biosensor transduction techniques and devices may be employed in the invention, future improvements in miniaturization and in other analytical techniques such as mass spectroscopy, gas chromatography, and NMR may allow such other techniques and systems to also be used in the invention. Furthermore, any of the techniques used in immunoassay systems, including colored latex beads, colloidal gold (provided, of course, that the gold does not interfere with the assay system), enzymes, fluorophores, etc. can be used in the practice of this invention.

### EXAMPLES

The following Examples are illustrative of the protein/DNA interaction and the binding affinity of the ArsR for arsenic.

### Example 1: Expression and Purification of E. coli ArsR

A synthetic gene for high level expression of ArsR from *E. coli* was prepared using codon optimization (Wada et al., Nucleic Acids Res. 1992, 20 (Suppl.):2111-2118; Zhang et al., Gene 1991, 105:61-72). Codon optimization, using triplet sequences that encode mRNA triplets that match with higher concentrations of tRNA and thus increase the efficiency of translation, was used to increase yield. The sequence of the gene, and protein it encodes, are shown in SEQ ID NOS. 1 and 2, respectively. Restriction site additions are present in the first three and last six bases of the sequence in SEQ ID NO:1.

For initial cell growth, a fresh transformation was first performed on Kan Plates overnight. Next, a culture from the colony pick was grown overnight in 50 ml of LB, 25 µg/ml Kanamycin for selection, at 37°C. The plasmid used was pET30A+ containing the optimized sequence for the ArsR protein (GenBank Accession# x 16045 Version-x16045.1 GI:42716) fused with a His6 carboxy terminal tag. This places the ATG of this sequence in frame with the first ATG of the vector and yields the sequence ....KAVCILEHHHHHH (SEQ ID NO: 11) at the carboxy terminal end of the protein, where the sequence KAVCI (SEQ ID NO:12) is from the ArsR protein, LE is from the Xho1 site, and the His6 tag is HHHHHH (SEQ ID NO:13) in the vector at the carboxy terminus. The gene sequence was inserted into the plasmid polylinker to utilize the T7 transcriptional control system. The transformed cells were BL21 DE3 (RecA⁻, F⁻, ompT, HSDS, RB⁻, MB⁻, gal, DCM, HMS174, available from Novagen, # 70235-3). One liter of LB + 1% Glucose, 25 ug/ml Kanamycin was inoculated and grown in shaker flask (250 rpm) at 37°C overnight.

For fermentor growth (5 liters size), 5 liters of LB+1% glycerol, 25 ug/ml Kanamycin, was inoculated with 500 ml of saturated cell growth (from the preceding culture), and agitated at 500 rpm, with air feed of 10,000 cc/min, at 37°C. The optical density was monitored at 600 nm. When the optical density was 1.0 (early/mid-log), the sample was induced with 0.5 mM IPTG after taking a pre-induction sample, and then grown post-induction for 3 hours. Cells were harvested by Sharples continuous centrifuge and re-suspended in PBS. The pellet was then washed, centrifuged and weighed. The resulting pellet weight was 35 g.

For extract production, a 1:4 wet pellet weight-to-volume re-suspension was produced in lysis buffer, which contained: 50 mM Hepes, pH 8.5, 0.5 M NaCl. Protease inhibitors (leupeptin, PMSF, Aprotin) were added. Extraction was accomplished by homogenization using a probe generator, with three pulses at 5 seconds each. The suspension was then nebulized for cell lysis (200 PSI, three passes) and spun at 50,000 x g for 45 minutes. The supernatant was then decanted.

For purification, chromatography (IMAC) was used. A 5 ml Hi-Trap IMAC column charged with NiCl in the following buffer A was equilibrated: 50 mM Hepes,
0.5 M NaCl. Soluble extract was then loaded in the column at 2 ml/min, washed to baseline with buffer A, and washed with 10 CV of A+ 10 mM imidizole. The column was then washed with 10 CV of A+ 20 mM Imidizole. Fractions (5 ml) were collected for SDS-PAGE analysis. The column was eluted with A+ 200 mM Imidizole, and fractions were collected into 20 mM DTT for SDS-PAGE analysis.

SDS-PAGE analysis was performed of the starting material, flow-through, wash, and elution. Pooling decisions were made and the protein concentration of pooled samples was determined.

Dialysis was performed next. Glycerol was added to bring the pool to a concentration of 5% glycerol by volume. The pool was then diluted to approximately 1 mg/ml with the dialysis buffer listed below. The faction pool was dialyzed in 3500 Molecular Weight Cutoff tubing placed in 1 liter consisting of 50 mM Hepes, pH 8.5, 200 mM NaCl, 1 mM EDTA, 15 mM DTT, and 5% glycerol for five hours. The dialysis tubing was then placed in 4 liters of 50 mM Hepes, pH 8.5, 200 mM NaCl, 15 mM DTT, and 5% glycerol overnight to remove any EDTA. The pool was then clarified by centrifugation, the protein concentration was determined, and a SDS-PAGE was run of the results.

The final product of a pilot production (5 g whole cell pellet from 34 g total) yielded a protein concentration of 480 µg/ml (in a 0.05% polysorbate solution for stabilization of the protein and reduction of non-specific binding to the sensor surface). The total volume for this experiment was 3.6 ml, which yielded a total protein yield of 1.75 mg. This was dissolved in a buffer of 50 mM Hepes, pH 8.5, 200 mM NaCl, 15 mM DTT, and 5% Glycerol. Purity was greater than 95% by SDS PAGE. In this system, induction was tightly regulated and successful, with 30-40% of protein production in soluble fraction. The initial yield from IMAC for dialysis was 5 mg at 1.0 mg/ml., with visible precipitation post dialysis. The post-dialysis yield was 1.75 mg at 0.48 mg/ml, 95% pure (see gel), and the protein was stable to multiple freeze thaw cycles.

Removing amino acids 98-117 or at least from the last cysteine to the carboxy end of the protein should make a protein less susceptible to aggregation/precipitation.

### Example 2: Immobilization of the DNA and Binding of ArsR to the DNA

Table 1 displays the sequences for the DNA used in the ArsR protein binding. The operon can be found on a plasmid or in the genome. Therefore, PLAS indicates the sequence from existing plasmid and CHROM indicates the chromosomal sequences on certain bacteria. The letter following this designation is either a L for long or an S for short. The following "1B" or "1T" designates top or bottom. Therefore, PLASL1T describes the top, long oligo sequence from plasmid.

**TABLE 2 DNA Sequences for ArsR Protein Binding**

| DNA | Sequence | SEQ ID NO: |
|---|---|---|
| PLASL1T | 5' Biotin -TTA ATC ATA TGC GTT TTT GGT TAT GTG TTG- | 3 |
| PLASL1B | 5' CAA CAC ATA ACC AAA AAC GCA TAT GAT T | 4 |
| CHROML1T | 5' Biotin- CTG CAC TTA CAC ATT CGT TAA GTC ATA TAT GTT TTT GAC TTA- | 5 |
| CHROML1B | 5' TAA GTC AAA AAC ATA TAT GAC TTA ACG AAT GTG TAA GTG C | 6 |
| PLASSIT | 5' Biotin- TTA ATC ATA TGC GTT TTT GGT TA- | 7 |
| PLASS1B | 5' TA ACC AAA AAC GCA TAT GAT T | 8 |
| CHROMS1T | 5' Biotin- TTA AGT CAT ATA TGT TTT TGA CTT A- | 9 |
| CHROMS1B | 5' T AAG TCA AAA ACA TAT ATG ACT TAA | 10 |

First, to immobilize the DNA, each biotinylated oligo was captured on a sensor surface pre-coated with streptavidin. The complimentary strand for each oligo was then injected over the surface to allow for hybridization of the DNA strands. A total of 1525 resonance units (RU) of dsDNA were immobilized on the sensor surface. A resonance unit (RU) is defined as the signal recorded to detect the change in the refractive index close to a chip surface. An increase of mass at the chip surface results in a change in the refractive index. A blank flowcell was used as a control surface in each experiment. The running buffer was HBS-EP (0.0 1 M HEPES pH 7.4, 0.15M Na Cl, 3mM EDTA, 0.005% Surfactant P20) and the instrument (BIAcore biosensor) temperature was 25°C.

The protein was diluted to various concentrations in the running buffer and injected over the DNA and control surfaces using an automated method. The sensorgrams were control subtracted and evaluated using BiaEvaluation software. The ArsR binding data for each of the DNA surfaces was detected. PLASL1 was immobilized on a separate sensor chip and the same binding data was collected for ArsR as described above. The protein binding and release rates, derived from the buildup and decrease respectively of the relative refractive index values, were analyzed for their relevant kinetic constants and used to calculate the protein-DNA binding affinity.

ArsR (3.27µM) was injected over PLASL1 and CHROML1 surfaces in the presence of various concentrations of phenylarsine oxide (PAO). At the highest concentration of PAO (100µM), ArsR binding was inhibited about 55%.

ArsR (3.27µM) was injected over PLASL1 and CHROML1 surfaces in the presence of various concentrations of sodium arsenate. The binding data for each DNA surface was obtained. The data from these experiments suggest that the protein-DNA binding affinities were different for each sequence used and that all sequence bound with a Kd of 0.5 uM ± 10 nM.

### Example 3: Prototype

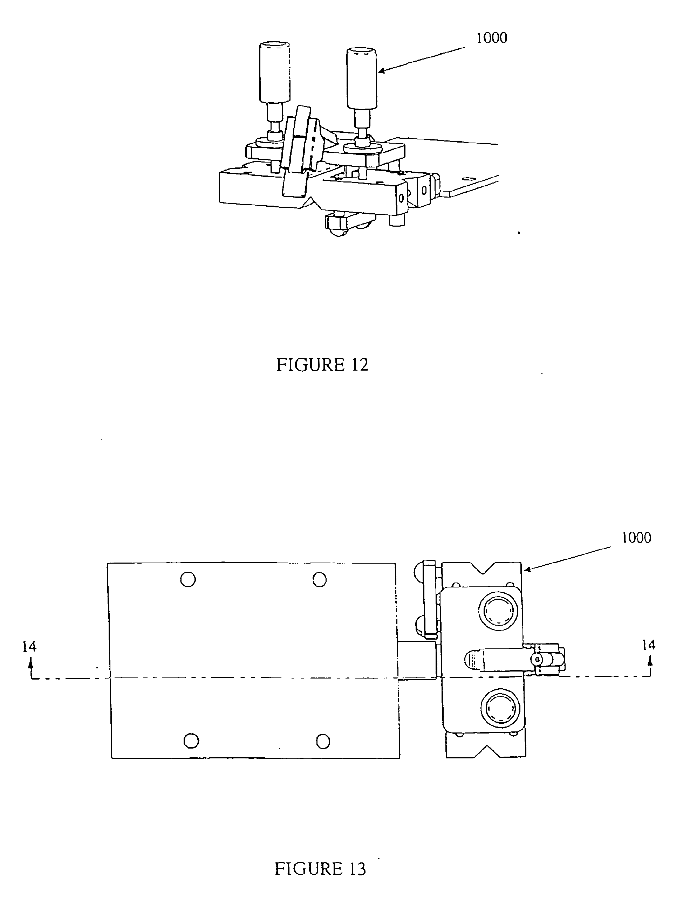

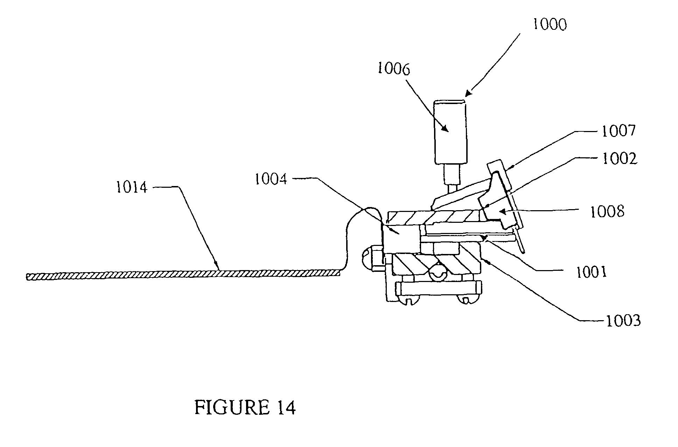

Figure 12 illustrates the top perspective view of the apparatus 1000 for maintaining the Spreeta^{™} 2000 (S2K) device 1001. Figure 13 illustrates the top view of the apparatus 1000. Figure 14 illustrates the cross-sectional side view of Figure 13 cut along line 14-14. One feature of the apparatus 1000 is the clamp 1002 and clamp block 1003 which firmly grasp the S2K device 1001 and holds it in appropriate relation to its mating connector 1004. When and if agitation is provided, the clamp prevents relative motion between the S2K device 1001 and the connector 1004. Instead, motion is absorbed in the interconnecting cable 1005. The clamp 1002 has some float in relation to the case. Tightening of the clamp 1002 to secure the S2K device 1001 can be by knurled thumb screws 1006, a 1.4 turn actuator, or a slide.

A flow cell 1007 allowing for injection of a quantity of fluid is affixed to the front surface of the S2K device 1001. As designed in this device, the flow cell 1007 attaches to the S2K device 1001 without the use of any hardware. It grasps the plastic protrusion on the side of the S2K device 1001 by fingers that fit within the recess of the device. Removal of the flow cell 1007 involves simple unsnapping of the grasping arms 1008 of the flow cell. A PC Board 1014 provides for data acquisition, analysis, and display.

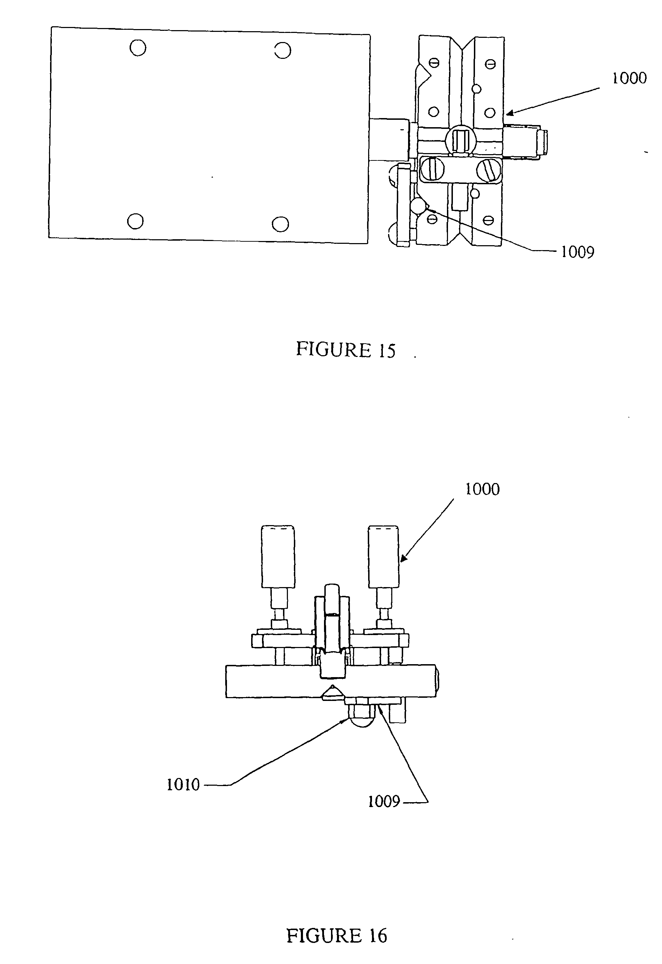

Figure 15 illustrates the bottom view of the apparatus 1000. An agitator 1009 for agitating fluid captured in the flow cell 1007 may be an eccentric motor system found in pagers and cell phones (illustrated here). Alternatively, a small audio speaker driven at the appropriate frequency for peak agitation may be used. Alternatively, a ferrous bead encapsulated in a benign coating and contained in the flow cell 1007 can be agitated by passing a magnet back and forth over the surface of the flow cell 1007.

Figure 16 illustrates the front view of the apparatus 1000. The agitator 1009 can be seen as held into place by the motor clamp 1010.

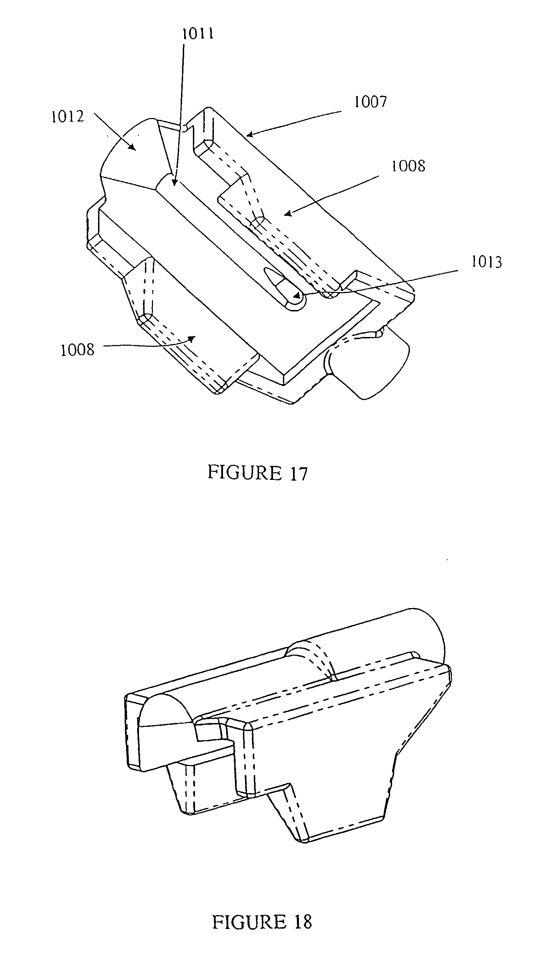

Figure 17 illustrates the bottom perspective view of the flow cell 1007. The flow cell 1007 when affixed to the front surface of the S2K device allows for the injection of a quantity of fluid. It will grasp the plastic protrusion on the side of the S2K device 1001 by fingers that fit within the recess of said device. The removal of the flow cell 1007 can be a simple unsnapping of the grasping arms 1008 of the flow cell. To ensure temperature equilibrium of fluid entering the flow cell 1007, the cell can be made of a thermally conductive material such as aluminum, appropriately coated for corrosion resistance. The entry path 1011 is long enough that the fluid, upon being introduced at an appropriate rate, can come to temperature equilibrium with the surrounding environment. The thermal mass of the aluminum flow cell 1007 would be much greater than the thermal mass of the fluid being brought to temperature.

An opening 1012 one end is sufficient to allow a pipette tip or hypodermic tip to be inserted and form a fluid tight seal. An opening 1013 in the far side of the flow cell is of a size sufficient to retain fluid due to surface tension alone. This will allow ejection of fluid automatically when new fluid is forced into the entry orifice and provide for fluid retention when no more fluid is being inserted.

The information given to the operator of the instrument in the preferred implementation shall be simple and easy to read. Output can be in the parts per billion of pollutant showing up on two or three character digital LCD readout. Device status information may be encoded into the digital segments or indicated by arrows on the LCD pointing to text printed on an overlay surrounding the display. These features make the instrument user friendly to the untrained operator.

## Claims

1. A system for detecting the presence of an analyte in a sample comprising
(i) an isolated protein that specifically binds the analyte
(ii) an isolated nucleic acid containing a specific binding sequence which is bound specifically by the protein, wherein specific binding of said analyte to said protein causes a change in the specific binding of said protein to said specific binding sequence; and
(iii)a detection system that indicates a change in binding of the protein to the nucleic acid in the presence of the analyte.

2. The system of claim 1, wherein the protein is a bacterial DNA-binding regulatory protein encoded by a metal-response operon and the nucleic acid is a DNA containing a specific binding sequence from the metal-response operon promoter that is specifically bound by the protein.

3. The system of claim 2, wherein the metal compound is As, the protein is ArsR, and the promoter is an *ars* operon promoter.

4. A biosensor device for detecting the presence of an analyte in a sample comprising
(i) a protein that specifically binds the analyte;
(ii) an isolated nucleic acid containing a specific binding sequence which is bound specifically by the protein wherein specific binding of said analyte to said protein causes a change in the specific binding of said protein to said specific binding sequence; and
(iii)a detection system that indicates a change in binding of the protein to the nucleic acid in the presence of the analyte; wherein either the protein or the nucleic acid is bound to a solid phase support.

5. The system of claim 1 or the device of claim 4, wherein protein binding to the nucleic acid and the analyte is mutually exclusive, such the protein bound to the nucleic acid is release from the nucleic acid upon binding the analyte.

6. The device of claim 4, wherein the detection system is a surface plasmon resonance system.

7. The device of claim 4, wherein the protein is a bacterial DNA-binding regulatory protein encoded by a metal-response operon and the nucleic acid is a DNA containing a specific binding sequence from the metal-response operon promoter that is specifically bound by the metal compound.

8. The device of claim 7, wherein the analyte is a metal compound selected from the group consisting of Ag, As, Cd, Cr, Hg, and Pb.

9. The device of claim 8, wherein the metal compound is As, the protein is ArsR, and the promoter is an *ars* operon promoter.

10. The device of claim 9, wherein the *ars* operon is an *E. coli ars* operon.

11. The device of claim 4, wherein the solid support is in contact with a flow cell that permits flow of liquid over the solid support.

12. The device of claim 11, wherein the solid support comprises a metal film that forms an interface with liquid, wherein the detection system is a surface plasmon resonance system.

13. The device of claim 12, wherein the solid support is a silicon chip of less than 1 cm square area.

14. The device of claim 12, which device is fabricated of a material of high heat capacity to ensure temperature stability when sample is applied.

15. The device of claim 12, further comprising a microprocessor capable of processing changes in plasmon resonance at the surface over time.

16. A method of detecting the presence of an analyte in a sample, which method comprises:
(i) contacting the sample with a protein that specifically binds the analyte, which protein is bound to an isolated nucleic acid containing a specific binding sequence for the protein, wherein binding of the protein to the analyte is mutually exclusive of binding to the nucleic acid; and
(ii) detecting release of the protein from the nucleic acid.

17. The system of claim 5 or the method according to claim 16, wherein the protein undergoes an allosteric change upon binding the analyte.

18. The system of claim 1 or the method according to claim 16, wherein the nucleic acid is a DNA.

19. The system of claim 1 or the method according to claim 18, wherein the analyte is a metal compound selected from the group consisting of Ag, As, Ba, Cd, Cr, Hg, Pb, and Se.

20. The system or method according to claim 19, wherein the metal compound is As.

21. The method according to claim 16, wherein the protein is a bacterial DNA- binding regulatory protein encoded by a metal-response operon and the nucleic acid is a DNA containing a specific binding sequence from the metal-response operon promoter that is specifically bound by the protein.

22. The system of claim 2 or the method according to claim 21, wherein the analyte is a metal compound selected from the group consisting of Ag, As, Cd, Cr, Hg, and Pb.

23. The method according to claim 16, wherein the protein comprises a sequence of a bacterial DNA-binding regulatory protein encoded by a metal-response operon sufficient to bind DNA and the metal.

24. The method according to claim 16, wherein the nucleic acid is a DNA containing a sequence that is modified to differ by at least one nucleotide from a specific binding sequence from a metal-response operon promoter that is specifically bound by the protein, and which DNA is specifically bound by the protein with different binding constants than the unmodified sequence.

## Patentansprüche

1. System zum Nachweis der Anwesenheit eines Analyten in einer Probe, umfassend:
(i) ein isoliertes Protein, das speziell den Analyten bindet,
(ii) eine isolierte eine spezifische speziell durch das Protein gebundene Bindesequenz enthaltende Nukleinsäure, wobei die spezifische Bindung des genannten Analyten an das genannte Protein eine Veränderung in der spezifischen Bindung des genannten Proteins an die genannte spezifische Bindesequenz verursacht, und
(iii) ein Nachweissystem, das eine Veränderung in der Bindung des Proteins an die Nukleinsäure in Gegenwart des Analyten anzeigt.

2. System nach Anspruch 1, wobei das Protein ein bakterielles, durch ein metallrespondierendes Operon kodiertes DNA-Binde- und Regulatorprotein und die Nukleinsäure eine eine spezifische Bindesequenz des metall-respondierenden, spezifisch durch das Protein gebundenen Operonpromotors enthaltende DNA ist.

3. System nach Anspruch 2, wobei die Metallverbindung As, das Protein ArsR und der Promotor ein Arsenresistenzoperon-Promotor ist.

4. Biosensor-Gerät zum Nachweis der Anwesenheit eines Analyten in einer Probe, umfassend:
(i) ein Protein, das speziell den Analyten bindet,
(ii) eine isolierte eine spezifische speziell durch das Protein gebundene Bindesequenz enthaltende Nukleinsäure, wobei die spezifische Bindung des genannten Analyten an das genannte Protein eine Veränderung in der spezifischen Bindung des genannten Proteins an die genannte spezifische Bindesequenz verursacht, und
(iii) ein Nachweissystem, das eine Veränderung in der Bindung des Proteins an die Nukleinsäure in Gegenwart des Analyten anzeigt, wobei entweder das Protein oder die Nukleinsäure an ein Festphasen-Trägermaterial gebunden wird.

5. System nach Anspruch 1 bzw. Gerät nach Anspruch 4, wobei sich eine Proteinbindung an die Nukleinsäure und an den Analyten gegenseitig ausschließen, so dass das an die Nukleinsäure gebundene Protein von der Nukleinsäure nach Bindung des Analyten freigegeben wird.

6. Gerät nach Anspruch 4, wobei das Nachweissystem ein Oberflächen-Plasmonresonanz-System ist.

7. Gerät nach Anspruch 4, wobei das Protein ein bakterielles, durch ein metallrespondierendes Operon kodiertes DNA-Binde- und Regulatorprotein und die Nukleinsäure eine eine spezifische Bindesequenz des metall-respondierenden, spezifisch durch die Metallverbindung gebundenen Operonpromotors enthaltende DNA ist.

8. Gerät nach Anspruch 7, wobei der Analyt eine Metallverbindung, ausgewählt aus der Gruppe bestehend aus Ag, As, Cd, Cr, Hg und Pb ist.

9. Gerät nach Anspruch 8, wobei die Metallverbindung As, das Protein ArsR und der Promotor ein Arsenresistenzoperon-Promotor ist.

10. Gerät nach Anspruch 9, wobei das Arsenresistenzoperon ein E. Coli Arsenresistenzoperon ist.

11. Gerät nach Anspruch 4, wobei das feste Trägermaterial in Kontakt mit einer Messzelle steht, die den Flüssigkeitsfluss über das feste Trägermaterial erlaubt.

12. Gerät nach Anspruch 11, wobei das feste Trägermaterial einen Metallfilm umfasst, der eine Schnittstelle mit der Flüssigkeit bildet, wobei das Nachweissystem ein Oberflächen-Plasmonresonanz-System ist.

13. Gerät nach Anspruch 12, wobei das feste Trägermaterial ein Silikonchip mit einer Quadratfläche von weniger als 1 cm ist.

14. Gerät nach Anspruch 12, wobei das Gerät aus einem Material mit hoher Wärmeleistung hergestellt ist, um sicherzustellen, dass die Temperatur bei Aufbringen der Probe stabil bleibt.

15. Gerät nach Anspruch 12, wobei das Gerät weiterhin einen Mikroprozessor enthält, der die Veränderungen in der Plasmonresonanz auf der Oberfläche im Zeitablauf verarbeiten kann.

16. Verfahren zum Nachweis der Anwesenheit eines Analyten in einer Probe, umfassend:
(i) Abklatschen der Probe mit einem speziell den Analyten bindenden Protein, das an eine isolierte, eine spezifische Bindesequenz für das Protein enthaltende Nukleinsäure gebunden ist, wobei sich die Bindung des Proteins an den Analyten und die Bindung an die Nukleinsäure gegenseitig ausschließen, und
(ii) Nachweis der Freigabe des Proteins durch die Nukleinsäure.

17. System nach Anspruch 5 bzw. Verfahren gemäss Anspruch 16, wobei das Protein nach Bindung des Analyten eine allosterische Veränderung erfährt.

18. System nach Anspruch 1 bzw. Verfahren gemäss Anspruch 16, wobei die Nukleinsäure eine DNA ist.

19. System nach Anspruch 1 bzw. Verfahren gemäss Anspruch 18, wobei der Analyt eine Metallverbindung, ausgewählt aus der Gruppe bestehend aus Ag, As, Ba, Cd, Cr, Hg, Pb und SE ist.

20. System bzw. Verfahren gemäss Anspurch 19, wobei die Metallverbindung As ist.

21. Verfahren gemäss Anspruch 16, wobei das Protein ein bakterielles, durch ein metallrespondierendes Operon kodiertes DNA-Binde- und Regulatorprotein und die Nukleinsäure eine eine spezifische Bindesequenz des metall-respondierenden, spezifisch durch das Protein gebundenen Operonpromotors enthaltende DNA ist.

22. System nach Anspruch 2 bzw. Verfahren gemäss Anspruch 21, wobei der Analyt eine Metallverbindung, ausgewählt aus der Gruppe bestehend aus Ag, As, Cd, Cr, Hg und Pb ist.

23. Verfahren gemäss Anspruch 16, wobei das Protein eine Sequenz aus einem bakteriellen DNA-Binde- und Regulatorproteins umfasst, das mit einem metallrespondierenden Operon kodiert ist, welches zur Bindung der DNA und des Metalls ausreicht.

24. Verfahren gemäss Anspruch 16, wobei die Nukleinsäure eine DNA ist, welche eine Sequenz enthält, die so modifiziert ist, dass sie sich mindestens in einem Nukleotid von einer spezifischen Bindesequenz aus einem metallrespondierenden, speziell durch das Protein gebundenen Operon unterscheidet, und die DNA spezifisch durch das Protein mit unterschiedlichen Bindungskonstanten als die unmodifizierte Sequenz gebunden wird.

## Revendications

1. Système pour détecter la présence d'une substance à analyser dans un échantillon comprenant
i) une protéine isolée qui se lie de façon spécifique à la substance à analyser
ii) un acide nucléique isolé comprenant une séquence de fixation spécifique à laquelle se lie spécifiquement la protéine, dans lequel la liaison spécifique de ladite substance à analyser à ladite protéine provoque une modification dans la fixation spécifique de ladite protéine à ladite séquence de fixation; et
iii) un système de détection qui indique une modification dans la fixation de la protéine à l'acide nucléique en présence de la substance à analyser.

2. Système de la revendication 1, dans lequel la protéine est une protéine bactérienne de régulation de la fixation à l'ADN codée par un opéron réponse-métal et l'acide nucléique est un ADN comprenant une séquence de fixation spécifique du promoteur de l'opéron réponse-métal auquel se fixe spécifiquement la protéine.

3. Système de la revendication 2, dans laquelle le composé métallique est l'As, la protéine est l'ArsR et le promoteur est un promoteur de l'opéron *ars.*

4. Dispositif biosenseur pour la détection de la présence d'une substance à analyser dans un échantillon comprenant
i) une protéine qui se fixe spécifiquement à la substance à analyser;
ii) un acide nucléique isolé qui contient une séquence de fixation spécifique à laquelle se fixe spécifiquement la protéine et dans laquelle la fixation spécifique de ladite substance à analyser à ladite protéine provoque une modification dans la fixation spécifique de ladite protéine à ladite séquence de fixation spécifique; et
iii) un système de détection qui indique une modification au niveau de la fixation de la protéine à l'acide nucléique en présence de la substance à analyser; dans lequel soit la protéine soit l'acide nucléique est fixé à un support en phase solide.

5. Système de la revendication 1 ou du dispositif revendiqué dans la revendication 4, dans lequel la fixation de la protéine à l'acide nucléique et à la substance à analyser est mutuellement exclusive, de sorte que la protéine fixée à l'acide nucléique est relâchée de l'acide nucléique lors de la fixation de la substance à analyser.

6. Dispositif de la revendication 4, dans lequel le système de détection est un système de surface plasmon résonance.

7. Dispositif de la revendication 4, dans lequel la protéine est une protéine bactérienne de régulation de la fixation à l'ADN codée par un opéron réponse-métal et l'acide nucléique est un ADN comprenant une séquence de fixation spécifique du promoteur de l'opéron réponse-métal auquel se fixe spécifiquement le composé métallique.

8. Dispositif de la revendication 7, dans lequel la substance à analyser est un composé métallique sélectionné à partir du groupe composé de Ag, As, Cd, Hg et Pb.

9. Dispositif de la revendication 8, dans lequel le composé métallique est l'As, la protéine est l'ArsR et le promoteur est le promoteur de l'opéron *ars.*

10. Dispositif de la revendication 9, dans lequel l'opéron *ars* est un opéron *ars* d'E. coli.

11. Dispositif de la revendication 4, dans lequel le support solide est en contact avec une cellule de flux qui permet le flux de liquide au-dessus du support solide.

12. Dispositif de la revendication 11, dans lequel le support solide comprend un film métallique qui forme une interface avec le liquide, dans lequel le système de détection est un système de surface plasmon résonance.

13. Dispositif de la revendication 12, dans lequel le support solide est une puce en silice de mois de 1 cm de surface.

14. Dispositif de la revendication 12, lequel dispositif est fabriqué de matériaux à résistance élevée à la chaleur pour assurer une stabilité de la température quand l'échantillon est appliqué.

15. Dispositif de la revendication 12, comprenant, en outre, un microprocesseur capable de traiter les modifications dans la résonance plasmon à la surface en fonction du temps.

16. Procédé pour la détection de la présence d'une substance à analyser dans un échantillon, lequel procédé comprend :
i) mettre en contact l'échantillon avec une protéine qui fixe de façon spécifique la substance à analyser, laquelle protéine est fixée à un acide nucléique isolé comprenant une séquence de fixation spécifique pour cette protéine, dans laquelle la fixation de la protéine à la substance à analyser est mutuellement exclusive par rapport à la fixation de l'acide nucléique; et
ii) la détection du relâchement de la protéine par l'acide nucléique.

17. Système de la revendication 5 ou procédé selon la revendication 16, dans lequel la protéine subit une modification allostérique suite à la fixation à la substance à analyser.

18. Système de la revendication 1 ou procédé selon la revendication 16, dans lequel l'ADN est un acide nucléique.

19. Système de la revendication 1 ou procédé selon la revendication 18, dans lequel la substance à analyser est un composé métallique sélectionné à partir d'un groupe comprenant l'Ag, l'As, le Ba, le Cd, le Cr, le Hg, le Pb et le Se.

20. Système ou procédé selon la revendication 19, dans lequel le composé métallique est l'As.

21. Procédé selon la revendication 16, dans lequel la protéine est une protéine bactérienne de régulation de la fixation à l'ADN codée par un opéron réponse-métal et l'acide nucléique est un ADN comprenant une séquence de fixation spécifique du promoteur de l'opéron réponse-métal auquel se fixe spécifiquement la protéine.

22. Système de la revendication 2 ou procédé selon la revendication 21, dans lequel la substance à analyser est un composé métallique sélectionné à partir d'un groupe comprenant l'Ag, l'As, le Cd, le Cr, le Hg et le Pb.

23. Procédé selon la revendication 16, dans lequel la protéine comprend une séquence une protéine bactérienne de régulation de la fixation à l'ADN codée par un opéron réponse-métal qui est suffisant pour fixer l'ADN et le métal.

24. Procédé selon la revendication 16, dans lequel l'acide nucléique est un ADN comprenant une séquence qui est modifiée pour différer par au moins un nucléotide d'une séquence de fixation spécifique provenant du promoteur d'un opéron métal-réponse auquel se fixe de façon spécifique la protéine et lequel ADN est fixé spécifiquement par la protéine avec des constantes de fixation différentes que la séquence non-modifiée.


### SEQUENCE LISTING

<110> Regenesis
   Laing, Lance
<120> BIOSENSOR FOR SMALL MOLECULE ANALYTES
<130> 4107/1L443-US1
<160> 13
<170> PatentIn version 3.1
<210> 1
   <211> 360
   <212> DNA
   <213> Escherichia coli
<400> 1
   catatgcttc aactgacccc gctgcagctg tttaaaaatc tctcagatga aactcgcctt     60
   ggtattgttc tgctgcttcg cgaaatgggc gagctgtgtg tatgtgacct gtgtatggcc    120
   ctggaccaat ctcaaccaaa aatttcgcgt catctggcta tgctccgcga atccggcatt    180
   ctcctcgatc gtaaacaggg caaatgggtg cattatcgtc tctctccgca tattccgtct    240
   tgggccgccc agatcattga acaggcatgg ctttcacagc aagatgatgt gcaggtgatc    300
   gcccgcaaac tggcctccgt taactgttct ggctcatcaa aagcagtttg catcctcgag    360
<210> 2
   <211> 117
   <212> PRT
   <213> Escherichia coli
<300>
   <308> GenBank / CAA 34168
   <309> 1994-09-07
   <313> (1)..(117)
<400> 2
   Met Leu Gln Leu Thr Pro Leu Gln Leu Phe Lys Asn Leu Ser Asp Glu
   1               5                   10                  15
   Thr Arg Leu Gly Ile Val Leu Leu Leu Arg Glu Met Gly Glu Leu Cys
               20                  25                  30
   Val Cys Asp Leu Cys Met Ala Leu Asp Gln Ser Gln Pro Lys Ile Ser
           35                  40                  45
   Arg His Leu Ala Met Leu Arg Glu Ser Gly Ile Leu Leu Asp Arg Lys
       50                  55                  60
   Gln Gly Lys Trp Val His Tyr Arg Leu Ser Pro His Ile Pro Ser Trp
   65                  70                  75                  80
   Ala Ala Gln Ile Ile Glu Gln Ala Trp Leu Ser Gln Gln Asp Asp Val
                   85                  90                  95
   Gln Val Ile Ala Arg Lys Leu Ala Ser Val Asn Cys Ser Gly Ser Ser
               100                 105                 110
   Lys Ala Val Cys Ile
           115
<210> 3
   <211> 31
   <212> DNA
   <213> Artificial Sequence
<220>
   <223> PLASL1T biotinylated oligo
<220>
   <221> misc_feature
   <222> (1)..(1)
   <223> where n is a biotin
<400> 3
   nttaatcata tgcgtttttg gttatgtgtt g 31
<210> 4
   <211> 28
   <212> DNA
   <213> Artificial Sequence
<220>.
   <223> PLASL1B biotinylated oligo
<400> 4
   caacacataa ccaaaaacgc atatgatt 28
<210> 5
   <211> 43
   <212> DNA
   <213> Artificial Sequence
<220>
   <223> CHROML1T biotinylated oligo
<220>
   <221> misc_feature
   <222> (1)..(1)
   <223> where n is a biotin
<400> 5
   nctgcactta cacattcgtt aagtcatata tgtttttgac tta 43
<210> 6
   <211> 40
   <212> DNA
   <213> Artificial Sequence
<220>
   <223> CHROML1B biotinylated oligo
<400> 6
   taagtcaaaa acatatatga cttaacgaat gtgtaagtgc 40
<210> 7
   <211> 24
   <212> DNA
   <213> Artificial Sequence
<220>
   <223> PLASS1T biotinylated oligo
<220>
   <221> misc_feature
   <222> (1).. (1)
   <223> where n is a biotin
<400> 7
   nttaatcata tgcgtttttg gtta 24
<210> 8
   <211> 21
   <212> DNA
   <213> Artificial Sequence
<220>
   <223> PLASS1B biotinylated oligo
<400> 8
   taaccaaaaa cgcatatgat t 21
<210> 9
   <211> 26
   <212> DNA
   <213> Artificial Sequence
<220>
   <223> CHROMS1T biotinylated oligo
<220>
   <221> misc_feature
   <222> (1)..(1)
   <223> where n is a biotin
<400> 9
   nttaagtcat atatgttttt gactta 26
<210> 10
   <211> 25
   <212> DNA
   <213> Artificial Sequence
<220>
   <223> CHROMS1B biotinylated oligo
<400> 10
   taagtcaaaa acatatatga cttaa 25
<210> 11
   <211> 13
   <212> PRT
   <213> Artificial Sequence
<220>
   <223> synthetic peptide
<400> 11
   Lys Ala Val Cys Ile Leu Glu His His His His His His
   1               5                   10
<210> 12
   <211> 5
   <212> PRT
   <213> Artificial Sequence
<220>
   <223> synthetic peptide
<400> 12
   Lys Ala Val Cys Ile
   1               5
<210> 13
   <211> 6
   <212> PRT
   <213> Artificial Sequence
<220>
   <223> His6 tag
<400> 13
   His His His His His His
   1               5